# EUROPÄISCHE PATENTSCHRIFT

(11) **EP 3 283 104 B1**
(45) Veröffentlichungstag und Bekanntmachung des Hinweises auf die Patenterteilung: **11.12.2024**
(21) Anmeldenummer: 16717129.7
(22) Anmeldetag: 15.04.2016
(51) Int. Cl.: A61K 39/12, A61K 39/00, A61K 35/76, A61K 35/768, A61P 35/00

(54) **ARENAVIREN ZUR ANWENDUNG BEI DER BEHANDLUNG EINES TUMORS BEIM MENSCHEN**
ARENAVIRUS FOR USE IN TREATMENT OF TUMORS IN HUMAN
ARENAVIRUS POUR L'UTILISATION DANS LE TRAITEMENT DES TUMEURS DANS DES HUMAINS

(30) Priorität: 17.04.2015 DE 102015207036
(43) Veröffentlichungstag der Anmeldung: 21.02.2018
(73) Patentinhaber: ABALOS THERAPEUTICS GmbH, 40225 Düsseldorf (DE)
(72) Erfinder: LANG, Karl, Sebastian, D-45149 Essen (DE); KALKAVAN, Halime, 38103 Memphis TN (US)
(74) Vertreter: Schiweck Weinzierl Koch Patentanwälte Partnerschaft mbB
(86) Internationale Anmeldenummer: PCT/EP2016/058347
(87) Internationale Veröffentlichungsnummer: WO 2016/166285

(56) Entgegenhaltungen:
- WO-A1-2006/008074
- WO-A1-2009/083210
- KALKAVAN HALIME ET AL: "Spatiotemporally restricted arenavirus replication induces immune surveillance and type I interferon-dependent tumour regression", NATURE COMMUNICATIONS, NATURE PUBLISHING GROUP, GB, vol. 8, 1 March 2017 (2017-03-01), XP002782694, ISSN: 2041-1723, DOI: 10.1038/NCOMMS14447 |
- H. KALKAVAN ET AL: "Supplementary figures Spatiotemporally restricted arenavirusreplication induces immune surveillance and type I interferon-dependent tumour regression.", NATURE COMMUNICATIONS, 1 March 2017 (2017-03-01), pages 1 - 14, XP055719032, Retrieved from the Internet <URL:https://www.nature.com/articles/ncomms14447#MOESM1838> [retrieved on 20200729], DOI: https://www.nature.com/articles/ncomms14447#MOESM1838
- KERI L. SCHADLER ET AL: "Immunosurveillance by Antiangiogenesis: Tumor Growth Arrest by T Cell-Derived Thrombospondin-1", CANCER RESEARCH, vol. 74, no. 8, 3 March 2014 (2014-03-03), US, pages 2171 - 2181, XP055515182, ISSN: 0008-5472, DOI: 10.1158/0008-5472.CAN-13-0094
- ANONYMOUS: "transgene Organismen -", LEXIKON DER BIOLOGIE, 1 January 1996 (1996-01-01), XP055719027, Retrieved from the Internet <URL:https://www.spektrum.de/lexikon/biologie/transgene-organismen/67248> [retrieved on 20200729]
- PERSEPHONE BORROW ET AL: "Inhibition of the Type I Interferon Antiviral Response During Arenavirus Infection", VIRUSES, vol. 2, no. 11, 5 November 2010 (2010-11-05), pages 2443 - 2480, XP055722271, DOI: 10.3390/v2112443
- YAMING WANG ET AL: "Timing and Magnitude of Type I Interferon Responses by Distinct Sensors Impact CD8 T Cell Exhaustion and Chronic Viral Infection", CELL HOST & MICROBE, vol. 11, no. 6, 1 June 2012 (2012-06-01), NL, pages 631 - 642, XP055724403, ISSN: 1931-3128, DOI: 10.1016/j.chom.2012.05.003
- MACAL, M ET AL: "Plasmacytoid Dendritic Cells Are Productively Infected and Activated through TLR-7 Early after Arenavirus Infection", CELL HOST & MICROBE, vol. 11, no. 6, 1 June 2012 (2012-06-01), NL, pages 617 - 630, XP055281788, ISSN: 1931-3128, DOI: 10.1016/j.chom.2012.04.017
- STACHURA PAWEL ET AL: "Arenaviruses: Old viruses present new solutions for cancer therapy", FRONTIERS IN IMMUNOLOGY, vol. 14, 24 March 2023 (2023-03-24), Lausanne, CH, XP093107879, ISSN: 1664-3224, DOI: 10.3389/fimmu.2023.1110522
- ADRIAN CIUREA: "Persistence of lymphocytic choriomeningitis virus at very low levels in immune mice", PROCEEDINGS OF THE NATIONAL ACADEMY OF SCIENCES, vol. 96, no. 21, 12 October 1999 (1999-10-12), pages 11964 - 11969, XP093161661, ISSN: 0027-8424, DOI: 10.1073/pnas.96.21.11964
- J. C. ZAPATA: "Lymphocytic choriomeningitis virus (LCMV) infection of macaques: A model for Lassa fever", ANTIVIRAL RESEARCH, 27 July 2011 (2011-07-27), pages 125 - 138, XP093161690, Retrieved from the Internet <URL:https://www.ncbi.nlm.nih.gov/pmc/articles/PMC4055450/> DOI: 10.1016/j.antiviral.2011.07.015
- DJAVANI, M. ET AL.: "Sequence comparison of the large genomic RNA segments of two strains of lymphocytic choriomeningitis virus differing in pathogenic potential for guinea pigs", VIRUS GENES, 1 January 1998 (1998-01-01), United States, pages 151 - 155, XP093164248, Retrieved from the Internet <URL:https://link.springer.com/article/10.1023/A:1008016724243> DOI: 10.1023/A:1008016724243
- STACI A. FISCHER ET AL: "Transmission of Lymphocytic Choriomeningitis Virus by Organ Transplantation", NEW ENGLAND JOURNAL OF MEDICINE, vol. 354, no. 21, 25 May 2006 (2006-05-25), pages 2235 - 2249, XP055078830, ISSN: 0028-4793, DOI: 10.1056/NEJMoa053240
- ELIZABETH KELLY ET AL: "History of Oncolytic Viruses: Genesis to Genetic Engineering InTroducTIon", 1 January 2007 (2007-01-01), pages 651 - 659, XP055281972, Retrieved from the Internet <URL:http://www.nature.com/mt/journal/v15/n4/pdf/6300108a.pdf> DOI: 10.1038/mt.sj.6300108
- N MOLOMUT ET AL: "Inhibition of transplantable and spontaneous murine tumours by the M-P virus.", NATURE, vol. 208, no. 5014, 4 December 1965 (1965-12-04), United Kingdom, pages 948 - 950, XP055282011, ISSN: 0028-0836
- WEBB H E ET AL: "The treatment of 18 cases of malignant disease with an arenavirus.", CLINICAL ONCOLOGY JUN 1975, vol. 1, no. 2, June 1975 (1975-06-01), pages 157 - 169, XP008180654, ISSN: 0305-7399
- REISEROVA L ET AL: "Identification of MaTu-MX Agent as a New Strain of Lymphocytic Choriomeningitis Virus (LCMV) and Serological Indication of Horizontal Spread of LCMV in Human Population", VIROLOGY, ELSEVIER, AMSTERDAM, NL, vol. 257, no. 1, 25 April 1999 (1999-04-25), pages 73 - 83, XP004440106, ISSN: 0042-6822, DOI: 10.1006/VIRO.1999.9638
- CHUPEI ZHANG ET AL: "Pseudotyping Lentiviral Vectors with Lymphocytic Choriomeningitis Virus Glycoproteins for Transduction of Dendritic Cells and In Vivo Immunization", HUMAN GENE THERAPY METHODS, vol. 25, no. 6, 1 December 2014 (2014-12-01), pages 328 - 338, XP055281098, ISSN: 1946-6536, DOI: 10.1089/hgtb.2014.105
- MILETIC ET AL: "77. Efficient Transduction and Therapy of Malignant Glioma by Lentiviral Vectors Pseudotyped with LCMV Glycoproteins", MOLECULAR THERAPY, NATURE PUBLISHING GROUP, GB, vol. 11, 15 August 2005 (2005-08-15), pages 31 - 32, XP005015416, ISSN: 1525-0016
- "Selective Transduction of Malignant Glioma by Lentiviral Vectors Pseudotyped with LCMV Glycoproteins", MOLECULAR THERAPY, NATURE PUBLISHING GROUP, GB, vol. 9, 1 May 2004 (2004-05-01), pages 374, XP004635343, ISSN: 1525-0016
- MASAHARU IWASAKI ET AL: "ABSTRACT", JOURNAL OF VIROLOGY., vol. 89, no. 23, 1 December 2015 (2015-12-01), US, pages 12166 - 12177, XP055281088, ISSN: 0022-538X, DOI: 10.1128/JVI.02075-15
- SANDRA ELIZABETH GO PRG A+-I ET AL: "Molecular analysis of the virulence attenuation process in Jun PRG A-n virus vaccine genealogy", VIRUS GENES, KLUWER ACADEMIC PUBLISHERS, BO, vol. 40, no. 3, 11 February 2010 (2010-02-11), pages 320 - 328, XP019795977, ISSN: 1572-994X
- SANDRA ELIZABETH GOÑI ET AL: "Genomic Features of Attenuated Junín Virus Vaccine Strain Candidate", VIRUS GENES, KLUWER ACADEMIC PUBLISHERS, BO, vol. 32, no. 1, 1 February 2006 (2006-02-01), pages 37 - 41, XP019216326, ISSN: 1572-994X, DOI: 10.1007/S11262-005-5843-2
- KOMA TAKAAKI ET AL: "Innate Immune Response to Arenaviral Infection: A Focus on the Highly Pathogenic New World Hemorrhagic Arenaviruses", JOURNAL OF MOLECULAR BIOLOGY, vol. 425, no. 24, 13 December 2013 (2013-12-13) - 13 December 2013 (2013-12-13), pages 4893 - 4903, XP028775401, ISSN: 0022-2836, DOI: 10.1016/J.JMB.2013.09.028
- ALLISON GROSETH ET AL: "Tacaribe Virus but Not Junin Virus Infection Induces Cytokine Release from Primary Human Monocytes and Macrophages", PLOS NEGLECTED TROPICAL DISEASES, vol. 5, no. 5, 10 May 2011 (2011-05-10), pages e1137, XP055282080, DOI: 10.1371/journal.pntd.0001137

### Sequenzprotokoll

### (SEQUENCE LISTING)

<110> Lang Karl Sebastian
<120> Arenaviren zur Anwendung bei der Therapie von Karzinomen oder Sarkomen sowie Verfahren zur Herstellung von Arenaviren mit einer verbesserten immuntherapeutischen Wirkung gegenüber Karzinomen oder Sarkomen
<130> P54975WO
<160> 6
<170> BiSSAP 1.3
<210> 1
<211> 3413
<212> RNA
<213> Junin virus
<220>
<223> Junin virus strain Candid-1 segment S - AY746353 - viruses; ssrna negative-strand viruses; arenaviridae; arenavirus; new world arenaviruses
<400> 1
   gtgcagtaag gggatcctag gcgattttgg ttacgctata attgtaactg ttttctgttt 60
   ggacaacatc aaaaacatcc attgcacaat ggggcagttc attagcttca tgcaagaaat 120
   accaaccttt ttgcaggagg ctctgaacat tgctcttgtt gcagtcagtc tcattgccat 180
   cattaagggt atagtgaact tgtacaaaag tggtttattc caattctttg tattcctagc 240
   gcttgcagga agatcctgca cagaagaagc tttcaaaatc ggactgcaca ctgagttcca 300
   gactgtgtcc ttctcaatgg tgggtctctt ttccaacaat ccacatgacc tacctttgtt 360
   gtgtacctta aacaagagcc atctttacat taaggggggc aatgcttcat ttcagatcag 420
   ctttgatgat attgcagtat tgttgccaca gtatgatgtt ataatacaac atccagcaga 480
   tatgagctgg tgttccaaaa gtgatgatca aatttggttg tctcagtggt tcatgaatgc 540
   tgtggggcat gattggcatc tagacccacc atttctgtgt aggaaccgtg caaagacaga 600
   aggcttcatc tttcaagtca acacctccaa gactggtgtc aatggaaatt atgctaagaa 660
   gtttaagact ggcatgcatc atttatatag agaatatcct gacccttgct tgaatggcaa 720
   actgtgctta atgaaggcac aacctaccag ttggcctctc caatgtccac tcgaccacgt 780
   taacacatta cacttcctta caagaggtaa aaacattcaa cttccaagga ggtccttgaa 840
   agcattcttc tcctggtctt tgacagactc atccggcaag gatacccctg gaggctattg 900
   tctagaagag tggatgctcg tagcagccaa aatgaagtgt tttggcaata ctgctgtagc 960
   aaaatgcaat ttgaatcatg actctgaatt ctgtgacatg ttgaggctct ttgattacaa 1020
   caaaaatgct atcaaaaccc taaatgatga aactaagaaa caagtaaatc tgatggggca 1080
   gacaatcaat gccctgatat ctgacaattt attgatgaaa aacaaaatta gggaactgat 1140
   gagtgtccct tactgcaatt acacaaaatt ttggtatgtc aaccacacac tttcaggaca 1200
   acactcatta ccaaggtgct ggttaataaa aaacaacagc tatttgaaca tctctgactt 1260
   ccgtaatgac tggatattag aaagtgactt cttaatttct gaaatgctaa gcaaagagta 1320
   ttcggacagg cagggtaaaa ctcctttgac tttagttgac atctgtattt ggagcacagt 1380
   attcttcaca gcgtcactct tccttcactt ggtgggtata ccctcccaca gacacatcag 1440
   gggcgaagca tgccctttgc cacacaggtt gaacagcttg ggtggttgca gatgtggtaa 1500
   gtaccccaat ctaaagaaac caacagtttg gcgtagagga cactaagacc tcctgagggt 1560
   ccccaccagc ccgggcactg cccgggctgg tgtggccccc cagtccgcgg cctggccgcg 1620
   gactggggag gcactgctta cagtgcatag gctgccttcg ggaggaacag caagctcggt 1680
   ggtaatagag gtgtaggttc ctcctcatag agcttcccat ctagcactga ctgaaacatt 1740
   atgcagtcta gcagagcaca gtgtggttca ctggaggcca acttgaaggg agtatccttt 1800
   tccctctttt tcttattgac aaccactcca ttgtgatatt tgcataagtg accatatttc 1860
   tcccagacct gttgatcaaa ctgcctggct tgttcagatg tgagcttaac atcaaccagt 1920
   ttaagatctc ttcttccatg gaggtcaaac aacttcctga tgtcatcgga tccttgagta 1980
   gtcacaacca tgtctggagg cagcaagccg atcacgtaac taagaactcc tggcattgca 2040
   tcttctatgt ccttcattaa gatgccgtga gagtgtctgc taccattttt aaaccctttc 2100
   tcatcatgtg gttttctgaa gcagtgaatg tactgcttac ctgcaggttg gaataatgcc 2160
   atctcaacag ggtcagtggc tggtccttca atgtcgagcc aaagggtgtt ggtggggtcg 2220
   agtttcccca ctgcctctct gatgacagct tcttgtatct ctgtcaagtt agccaatctc 2280
   aaattctgac cgtttttttc cggctgtcta ggaccagcaa ctggtttcct tgtcagatca 2340
   atacttgtgt tgtcccatga cctgcctgtg atttgtgatc tagaaccaat ataaggccaa 2400
   ccatcgccag aaagacaaag tttgtacaaa aggttttcat aaggatttct attgcctggt 2460
   ttctcatcaa taaacatgcc ttctcttcgt ttaacctgaa tggttgattt tatgagggaa 2520
   gagaagtttt ctggggtgac tctgattgtt tccaacatgt ttccaccatc aagaatagat 2580
   gctccagcct ttactgcagc tgaaagactg aagttgtaac cagaaatatt gatggagctt 2640
   tcatctttag tcacaatctg aaggcagtca tgttcctgag tcagtctgtc aaggtcactt 2700
   aagtttggat acttcacagt gtatagaagc ccaagtgagg ttaaagcttg tatgacactg 2760
   ttcattgtct cacctccttg aacagtcatg catgcaattg tcaatgcagg aacagagcca 2820
   aactgattgt ttagctttga agggtcttta acatcccata tcctcaccac accatttccc 2880
   ccagtccctt gctgttgaaa tcccagtgtt ctcaatatct ctgatctttt agcaagttgt 2940
   gactgggaca agttacccat gtaaaccccc tgagagcctg tctctgctct tcttatcttg 3000
   ttttttaatt tctcaaggtc agacgccaac tccatcagtt catccctccc cagatctccc 3060
   accttgaaaa ctgtgtttcg ttgaacactc ctcatggaca tgagtctgtc aacctcttta 3120
   ttcaggtccc tcaacttgtt gaggtcttct tccccctttt tagtctttct gagtgcccgc 3180
   tgcacctgtg ccacttggtt gaagtcgatg ctgtcagcaa ttagcttggc gtccttcaaa 3240
   acatctgact tgacagtctg agtgaattgg ctcaaacctc tccttaagga ctgagtccat 3300
   ctaaagcttg gaacctcctt ggagtgtgcc atgccagaag ttctggtgat tttgatctag 3360
   aatagagttg ctcagtgaaa gtgttagaca ctatgcctag gatccactgt gcg 3413
<210> 2
<211> 7114
<212> RNA
<213> Junin virus
<220>
<223> Junin virus strain Candid #1 segment L - AY819707 - viruses; ssrna negative-strand viruses; arenaviridae; arenavirus; new world arenaviruses
<400> 2
   cgcacagtgg atcctaggcg taacttcatc attaaaatct cagattctgc tctgagtgtg 60
   acttactgcg aagaggcaga caaatgggca actgcaacgg ggcatccaag tctaaccagc 120
   cagactcctc aagagccaca cagccagccg cagaatttag gagggtagct cacagcagtc 180
   tatatggtag atataactgt aagtgctgct ggtttgctga taccaatttg ataacctgta 240
   atgatcacta cctttgttta aggtgccatc agggtatgtt aaggaattca gatctctgca 300
   atatctgctg gaagcccctg cccaccacaa tcacagtacc ggtggagcca acagcaccac 360
   caccataggc agactgcaca gggtcagacc cgaccccccg gggggccccc atggggaccc 420
   cccgtggggg aaccccgggg gtgatgcgcc attagtcaat gtctttgatc tcgactttgt 480
   gcttcagtgg cctgcatgtc acccctttca atctgaactg cccttgggga tctgatatca 540
   gcaggtcatt taaagatctg ctgaatgcca ccttgaaatt tgagaattcc aaccagtcac 600
   caaatttatc aagtgaacgg atcaactgct ctttgtgtag atcataaacg aggacaaagt 660
   cctcttgctg aaataatatt gtttgtgatg ttgtttttag ataaggccat agttggctta 720
   ataaggtttc cacactatca atgtcctcta gtgctccaat tgccttgact atgacatccc 780
   cagacaactc aactctatat gttgacaacc tttcattacc tctgtaaaag ataccctctt 840
   tcaagacaag aggttctcct gggttatctg gcccaatgag gtcatatgca tacttgttac 900
   ttagttcaga ataaaagtca ccaaagttga acttaacatg gctcagaata ttgtcatcat 960
   ttgtcgcagc gtagcctgca tcaataaaca agccagctag gtcaaagctc tcatggcctg 1020
   tgaacaatgg taggctagcg ataaccagtg caccatccaa caatgagtgg cttccctcag 1080
   acccagaaac acattgactc attgcatcca cattcagctc taattcaggg gtaccgacat 1140
   catccactcc tagtgaactg acaatggtgt aactgtacac catctttctt ctaagtttaa 1200
   attttgtcga aactcgtgtg tgttctactt gaatgatcaa ttttagtttc acagcttctt 1260
   ggcaagcaac attgcgcaac acagtgtgca ggtccatcat gtcttcctga ggcaacaagg 1320
   agatgttgtc aacagagaca ccctcaagga aaaccttgat attatcaaag ctagaaacta 1380
   cataacccat tgcaatgtct tcaacaaaca ttgctcttga tactttatta ttcctaactg 1440
   acaaggtaaa atctgtgagt tcagctagat ctacttgact gtcatcttct agatctagaa 1500
   cttcattgaa ccaaaagaag gatttgagac acgatgttga catgactagt gggtttatca 1560
   tcgaagataa gacaacttgc accatgaagt tcctgcaaac ttgctgtggg ctgatgccaa 1620
   cttcccaatt tgtatactct gactgtctaa catgggctga agcgcaatca ctctgtttca 1680
   caatataaac attattatct cttactttca ataagtgact tataatccct aagttttcat 1740
   tcatcatgtc tagagccaca cagacatcta gaaacttgag tcttccacta tccaaagatc 1800
   tgttcacttg aagatcattc ataaagggtg ccaaatgttc ttcaaatagt ttggggtaat 1860
   ttcttcgtat agaatgcaat acatggttca tgcctaattg gtcttctatc tgtcgtactg 1920
   ctttgggttt aacagcccag aagaaattct tattacataa gaccagaggg gcctgtggac 1980
   tcttaatagc aaaaaacacc cactccccta actcacaggc atttgtcagc accaaagaga 2040
   agtaatccca caaaattggt ttagaaaatt ggttaacttc tttaagtgat ttttgacagt 2100
   aaataacttt aggctttctc tcacaaattc cacaaagaca tggcattatt cgagtaaata 2160
   tgtcctttat atacagaaat ccgcctttac catccctaac acacttactc cccatactct 2220
   tacaaaaccc aatgaagcct gaggcaacag aagactgaaa tgcagatttg ttgattgact 2280
   ctgccaagat cttcttcacg ccttttgtga aatttcttga cagcctggac tgtattgtcc 2340
   ttatcaatgt tggcatctct tctttctcta acactcttcg acttgtcatg agtttggtcc 2400
   tcaagaccaa cctcaagtcc ccaaagctcg ctaaattgac ccatctgtag tctagagttt 2460
   gtctgatttc atcttcacta cacccggcat attgcaggaa tccggataaa gcctcatccc 2520
   ctcccctgct tatcaagttg ataaggtttt cctcaaagat tttgcctctc ttaatgtcat 2580
   tgaacacttt cctcgcgcag ttccttataa acattgtctc cttatcatca gaaaaaatag 2640
   cttcaatttt cctctgtaga cggtaccctc tagacccatc aacccagtct ttgacatctt 2700
   gttcttcaat agctccaaac ggagtctctc tgtatccaga gtatctaatc aattggttga 2760
   ctctaatgga aatctttgac actatatgag tgctaacccc attagcaata cattgatcac 2820
   aaattgtgtc tatggtctct gacagttgtg ttggagtttt acacttaacg ttgtgtagag 2880
   cagcagacac aaacttggtg agtaaaggag tctcttcacc catgacaaaa aatcttgact 2940
   taaactcagc aacaaaagtt cctatcacac tctttgggct gataaacttg tttaatttag 3000
   aagataagaa ttcatggaag cacaccattt ccagcagttc tgtcctgtct tgaaactttt 3060
   catcactaag gcaaggaatt tttataaggc taacctggtc atcgctggag gtataagtga 3120
   caggtatcac atcatacaat aagtcaagtg cataacacag aaattgttca gtaattagcc 3180
   catataaatc tgatgtgttg tgcaagattc cctggcccat gtccaagaca gacattatat 3240
   ggctggggac ctggtccctt gactgcagat actggtgaaa aaactcttca ccaacactag 3300
   tacagtcaca acccattaaa cctaaagatc tcttcaattt ccctacacag taggcttctg 3360
   caacattaat tggaacttca acgaccttat gaagatgcca tttgagaatg ttcattactg 3420
   gttcaagatt cacctttgtt ctatctctgg gattcttcaa ttctaatgtg tacaaaaaag 3480
   aaaggaaaag tgctgggctc atagttggtc cccatttgga gtggtcatat gaacaggaca 3540
   agtcaccatt gttaacagcc attttcatat cacagattgc acgttcgaat tccttttctg 3600
   aattcaagca tgtgtatttc attgaactac ccacagcttc tgagaagtct tcaactaacc 3660
   tggtcatcag cttagtgttg aggtctccca catacagttc tctatttgag ccaacctgct 3720
   ccttataact tagtccaaat ttcaagttcc ctgtatttga gctgatgctt gtgaactctg 3780
   taggagagtc gtctgaatag aaacataaat tccgtagggc tgcatttgta aaataacttt 3840
   tgtctagctt atcagcaatg gcttcagaat tgctttccct ggtactaagc cgaacctcat 3900
   cctttagtct cagaacttca ctggaaaagc ccaatctaga tctacttcta tgctcataac 3960
   tacccaattt ctgatcataa tgtccttgaa ttaaaagata cttgaagcat tcaaagaatt 4020
   catcttcttg gtaggctatt gttgtcaaat tttttaataa caaacccaaa gggcagatgt 4080
   cctgcggtgc ttcaagaaaa taagtcaatt taaatggaga tagataaaca gcatcacata 4140
   actctttata cacatcagac ctgagcacat ctggatcaaa atccttcacc tcatgcattg 4200
   acacctctgc tttaatctct ctcaacactc caaaaggggc ccacaatgac tcaagagact 4260
   ctcgctcatc aacagatgga ttttttgatt tcaacttggt gatctcaact tttgtcccct 4320
   cactattagc catcttggct agtgtcattt gtacgtcatt tctaataccc tcaaaggccc 4380
   ttacttgatc ctctgttaaa ctctcataca tcactgataa ttcttcttga ttggttctgg 4440
   ttcttgaacc ggtgctcaca agacctgtta gattttttaa tattaagtag tccatggaat 4500
   caggatcaag attatacctg ccttttgttt taaacctctc agccatagta gaaacgcatg 4560
   ttgaaacaag tttctcctta tcataaacag aaagaatatt tccaagttcg tcgagcttgg 4620
   ggattaccac acttttattg cttgacagat ccagagctgt gctagtgatg ttaggcctgt 4680
   agggattgct tttcagttca cctgtaactt taagtcttcc tctattgaag agagaaatgc 4740
   agaaggacaa aatctcttta cacactcctg gaatttgagt atctgaggaa gtcttagcct 4800
   ctttggaaaa gaatctgtcc aatcctctta tcatggtgtc ctcttgttcc agtgttagac 4860
   tcccacttag aggggggttt acaacaacac aatcaaactt gactttgggc tcaataaact 4920
   tctcaaaaca cttgatttga tctgtcaggc gatcaggtgt ctctttggtt accaagtgac 4980
   acagataact aacatttaat agatatttaa accttcttgc aaagtaaaga tctgcatctt 5040
   ccccttcacc caaaattgtc tggaaaagtt ccacagccat cctctgaatc agcacctctg 5100
   atccagacat gcagtcgacc cttaactttg acatcaaatc cacatgatgg atttgatttg 5160
   catatgccat caagaaatat cttagacctt gtaaaaatgt ctggttcctt ttggaagggg 5220
   aacagagtac agctaacact aacaatctta atattggcct tgtcattgtc atgagttcgt 5280
   ggctaaaatc caaccagctg gtcatttcct cacacatttc aattaacaca tcctccgaaa 5340
   atataggcag gaaaaatctc tttggatcac agtaaaaaga gccttgttct tccaataccc 5400
   cattgatgga tagatagata gaatagcacc ttgacttctc acctgttttt tggtaaaaca 5460
   agagaccaaa tgtattcttt gtcagatgaa atctttgtac ataacactct cttagtctaa 5520
   cattcccaaa atatctagaa tactctcttt cattgattaa caatcgggag gaaaatgatg 5580
   tcttcatcga gttgaccaat gcaagggaaa tggaggacaa aatcctaaat aatttcttct 5640
   gctcaccttc cactaagctg ctgaatggct gatgtctaca gattttctca aattccttgt 5700
   taatagtata tctcatcact ggtctgtcag aaacaagtgc ctgagctaaa atcatcaagc 5760
   tatccatatc agggtgtttt attagttttt ccagctgtga ccagagatct tgatgagagt 5820
   tcttcaatgt tctggaacac gcttgaaccc acttggggct ggtcatcaat ttcttcctta 5880
   ttagtttaat cgcctccaga atatctagaa gtctgtcatt gactaacatt aacatttgtc 5940
   caacaactat tcccgcattt cttaacctta caattgcatc atcatgcgtt ttgaaaagat 6000
   cacaaagtaa attgagtaaa actaagtcca gaaacagtaa agtgtttctc ctggtgttga 6060
   aaacttttag acctttcact ttgttacaca cggaaagggc ttgaagataa cacctctcta 6120
   cagcatcaat agatatagaa ttctcatctg actggctttc catgttgact tcatctattg 6180
   gatgcaatgc gatagagtag actacatcca tcaacttgtt tgcacaaaaa gggcagctgg 6240
   gcacatcact gtctttgtgg cttcctaata agatcaagtc atttataagc ttagactttt 6300
   gtgaaaattt gaatttcccc aactgcttgt caaaaatctc cttcttaaac caaaacctta 6360
   actttatgag ttcttctctt atgacagatt ctctaatgtc tcctctaacc ccaacaaaga 6420
   gggattcatt taacctctca tcataaccca aagaattctt tttcaagcat tcgatgtttt 6480
   ctaatcccaa gctctggttt tttgtgttgg acaaactatg gatcaatcgc tggtattctt 6540
   gttcttcaat attaatctct tgcataaatt ttgatttctt taggatgtcg atcagcaacc 6600
   accgaactct ttcaacaacc caatcagcaa ggaatctatt gctgtagcta gatctgccat 6660
   caaccacagg aaccaacgta atccctgccc ttagtaggtc ggactttagg tttaagagct 6720
   ttgacatgtc actcttccat tttctctcaa actcatcagg attgacccta acaaaggttt 6780
   ccaataggat gagtgttttc cctgtgagtt tgaagccatc cggaatgact tttggaaggg 6840
   tgggacatag tatgccatag tcagacagga tcacatcaac aaacttctga tctgaattga 6900
   tctgacaggc gtgtgcctca caggactcaa gctctactaa acttgacaga agtttgaacc 6960
   cttccaacaa cagagagctg gggtgatgtt gagataaaaa gatgtccctt tggtatgcta 7020
   gctcctgtct ttctggaaaa tgctttctaa taaggctttt tatttcattt actgattcct 7080
   ccatgctcaa gtgccgccta ggatccactg tgcg 7114
<210> 3
<211> 3410
<212> RNA
<213> Junin mammarenavirus
<220>
<223> Junin virus strain Candid #1 segment S, completesequence.-ACCESSION FJ969442-VERSION FJ969442.1-GI:264665554 - Viruses; ssRNA viruses; ssRNA negative-strand viruses; Arenaviridae; Mammarenavirus.
<400> 3
   cgcacagtgg atcctaggcg attttggtta cgctataatt gtaactgttt tctgtttgga 60
   caacatcaaa aacatccatt gcacaatggg gcagttcatt agcttcatgc aagaaatacc 120
   aacctttttg caggaggctc tgaacattgc tcttgttgca gtcagtctca ttgccatcat 180
   taagggtgta gtgaacctgt acaaaagtgg tttgttccaa ttctttgtat tcctagcact 240
   cgcaggaaga tcctgcacag aagaagcttt taaaatcgga ctgcacacag agttccagac 300
   tgtgtccttc tcaatggtgg gtctcttttc caacaatcca catgacctgc ctctgttgtg 360
   taccttaaac aagagccatc tttacattaa ggggggcaat gcttcatttc agatcagctt 420
   tgatgatatt gcagtattgt tgccacagta tgatgttata atacaacatc cagcagatat 480
   gagctggtgt tccaaaagtg atgatcaaat ttggttgtct cagtggttca tgaatgctgt 540
   gggacatgat tggcatctag acccaccatt tctgtgtagg aaccgtgcaa agacagaagg 600
   cttcatcttt caagtcaaca cctccaagac tggtgtcaat ggaaattatg ctaagaagtt 660
   taagactggc atgcatcatt tatatagaga atatcctgac ccttgcttga atggcaaact 720
   gtgcttaatg aaggcacaac ctaccagttg gcctctccaa tgtccactcg accacgttaa 780
   cacattacac ttccttacaa gaggtaaaaa cattcaactt ccaaggaggt ccttgaaagc 840
   attcttctcc tggtctttga cagactcatc cggcaaggat acccctggag gctattgtct 900
   agaagagtgg atgctcgttg cagccaaaat gaagtgtttt ggcaatactg ctgtagcaaa 960
   atgcaatctg aatcatgact ctgaattctg tgacatgctg aggctttttg attacaacaa 1020
   aaatgctatc aaaaccttaa atgatgaaac taagaaacaa gtaaatctga tgggacagac 1080
   aatcaatgcg ctgatatctg acaatttatt gatgaaaaac aaaattaggg aactgatgag 1140
   tgtcccttac tgcaattaca caaaattttg gtatgtcaac cacacacttt caggacaaca 1200
   ctcattacca aggtgctggt taataaaaaa caacagctat ttgaacatct ctgacttccg 1260
   taatgactgg atattagaaa gtgacttctt aatttctgaa atgctaagca aagagtattc 1320
   ggacaggcag ggtaaaactc ctttgacttt agttgacatc tgtatttgga gcacagtatt 1380
   cttcacagcg tcactcttcc ttcacttggt gggtataccc tcccacagac acatcagggg 1440
   cgaagcatgc cctttgccac acaggttgaa cagcttgggt ggttgcagat gtggtaagta 1500
   ccccaatcta aagaaaccaa cagtttggcg tagaggacac taagacctcc tgagggtccc 1560
   caccagcccg ggcactgccc gggctggtgt ggccccccag tccgcggcct ggccgcggac 1620
   tggggaggca ctgcttacag tgcataggct gccttcggga ggaacagcaa gctcggtggt 1680
   aatagaggtg taggttcctc ctcatagagc ttcccatcta gcactgactg aaacattatg 1740
   cagtctagca gagcacagtg tggttcactg gaggccaact tgaagggagt atccttttcc 1800
   ctctttttct tattgacaac cactccattg tgatatttgc ataagtgacc atatttctcc 1860
   cagacctgtt gatcaaactg cctggcttgt tcagatgtga gcttaacatc aaccagttta 1920
   agatctcttc ttccatggag gtcaaacaac ttcctgatgt catcggatcc ttgagtagtc 1980
   acaaccatgt ctggaggcag caagccgatc acgtaactaa gaactcctgg cattgcatct 2040
   tctatgtctt tcattaagat gccgtgagag tgtctgctac catttttaaa ccctttctca 2100
   tcatgtggtt ttctgaagca gtgaatatac tgcttacctg caggctggaa caacgccatc 2160
   tcaacagggt cagtagctgg tccttcaatg tcgagccaaa gggtattggt ggggtcgagt 2220
   ttccccactg cctctctgat gacagcttct tgtatctctg tcaagttagc caatctcaaa 2280
   ttctgaccgt tcttttccgg ttgtctaggt ccagcaactg gtttccttgt cagatcaata 2340
   cttgtgttgt cccatgacct gcctatgatt tgtgatctgg aaccaatata aggccaacca 2400
   tcgccagaaa ggcaaagttt gtacagaagg ttttcataag ggtttctatt gcctggtttc 2460
   tcatcaataa acatgccttc tcttcgttta acctgaatgg ttgattttat gagggaagaa 2520
   aagttatctg gggtgactct gattgtctcc aacatatttc caccatcaag aatggatgca 2580
   ccagccttta ctgcagctga aagactaaag ttgtagccag aaatgttgat ggagctttca 2640
   tccttagtca caatctggag gcagtcatgt tcctgagtca atctgtcaag gtcactcaag 2700
   tttggatact tcacagtgta tagaagccca agagaggtta aagcctgtat gacactgttc 2760
   attgtctcac ctccttgaac agtcatgcat gcaattgtca atgcaggaac agaaccaaac 2820
   tgattgttaa gttttgaagg atctttaaca tcccataccc tcaccacacc atttccccca 2880
   gttccttgct gttgaaatcc cagtgttctc aatatctctg atctcttggc cagttgtgat 2940
   tgagacaagt tacccatgta aaccccttga gagcctgtct ctgctcttct aatcttgttt 3000
   tttaatttct caaggtcaga cgccaactcc atcagttcat ccctccccag atctcccacc 3060
   ttgaaaactg tgtttcgttg aacactcctc atggacatga gtctgtcaac ctctttattc 3120
   aggtccctca acttattgag gtcttcttcc ccccttttag tctttctgag tgcccgctgc 3180
   acctgtgcct cttggttgaa gtcaatgctg tcagcaatta gcttggcatc cttcagaaca 3240
   tccgacttga cagtctgagt aaattgactc aaacctctcc ttaaggactg agtccatcta 3300
   aagcttggaa cctctttgga gtgtgccatg ccagaagttc tggtgatttt gatctagaat 3360
   agagttgctc agtgaaagtg ttagacacta tgcctaggat ccactgtgcg 3410
<210> 4
<211> 7114
<212> RNA
<213> Junin mammarenavirus
<220>
<223> Junin virus strain Candid #1 segment L, complete sequence.-ACCESSION AY819707-VERSION AY819707.2-GI:227957900-Viruses; ssRNA viruses; ssRNA negative-strand viruses; Arenaviridae; Mammarenavirus.
<400> 4
   cgcacagtgg atcctaggcg taacttcatc attaaaatct cagattctgc tctgagtgtg 60
   acttactgcg aagaggcaga caaatgggca actgcaacgg ggcatccaag tctaaccagc 120
   cagactcctc aagagccaca cagccagccg cagaatttag gagggtagct cacagcagtc 180
   tatatggtag atataactgt aagtgctgct ggtttgctga taccaatttg ataacctgta 240
   atgatcacta cctttgttta aggtgccatc agggtatgtt aaggaattca gatctctgca 300
   atatctgctg gaagcccctg cccaccacaa tcacagtacc ggtggagcca acagcaccac 360
   caccataggc agactgcaca gggtcagacc cgaccccccg gggggccccc atggggaccc 420
   cccgtggggg aaccccgggg gtgatgcgcc attagtcaat gtctttgatc tcgactttgt 480
   gcttcagtgg cctgcatgtc acccctttca atctgaactg cccttgggga tctgatatca 540
   gcaggtcatt taaagatctg ctgaatgcca ccttgaaatt tgagaattcc aaccagtcac 600
   caaatttatc aagtgaacgg atcaactgct ctttgtgtag atcataaacg aggacaaagt 660
   cctcttgctg aaataatatt gtttgtgatg ttgtttttag ataaggccat agttggctta 720
   ataaggtttc cacactatca atgtcctcta gtgctccaat tgccttgact atgacatccc 780
   cagacaactc aactctatat gttgacaacc tttcattacc tctgtaaaag ataccctctt 840
   tcaagacaag aggttctcct gggttatctg gcccaatgag gtcatatgca tacttgttac 900
   ttagttcaga ataaaagtca ccaaagttga acttaacatg gctcagaata ttgtcatcat 960
   ttgtcgcagc gtagcctgca tcaataaaca agccagctag gtcaaagctc tcatggcctg 1020
   tgaacaatgg taggctagcg ataaccagtg caccatccaa caatgagtgg cttccctcag 1080
   acccagaaac acattgactc attgcatcca cattcagctc taattcaggg gtaccgacat 1140
   catccactcc tagtgaactg acaatggtgt aactgtacac catctttctt ctaagtttaa 1200
   attttgtcga aactcgtgtg tgttctactt gaatgatcaa ttttagtttc acagcttctt 1260
   ggcaagcaac attgcgcaac acagtgtgca ggtccatcat gtcttcctga ggcaacaagg 1320
   agatgttgtc aacagagaca ccctcaagga aaaccttgat attatcaaag ctagaaacta 1380
   cataacccat tgcaatgtct tcaacaaaca ttgctcttga tactttatta ttcctaactg 1440
   acaaggtaaa atctgtgagt tcagctagat ctacttgact gtcatcttct agatctagaa 1500
   cttcattgaa ccaaaagaag gatttgagac acgatgttga catgactagt gggtttatca 1560
   tcgaagataa gacaacttgc accatgaagt tcctgcaaac ttgctgtggg ctgatgccaa 1620
   cttcccaatt tgtatactct gactgtctaa catgggctga agcgcaatca ctctgtttca 1680
   caatataaac attattatct cttactttca ataagtgact tataatccct aagttttcat 1740
   tcatcatgtc tagagccaca cagacatcta gaaacttgag tcttccacta tccaaagatc 1800
   tgttcacttg aagatcattc ataaagggtg ccaaatgttc ttcaaatagt ttggggtaat 1860
   ttcttcgtat agaatgcaat acatggttca tgcctaattg gtcttctatc tgtcgtactg 1920
   ctttgggttt aacagcccag aagaaattct tattacataa gaccagaggg gcctgtggac 1980
   tcttaatagc aaaaaacacc cactccccta actcacaggc atttgtcagc accaaagaga 2040
   agtaatccca caaaattggt ttagaaaatt ggttaacttc tttaagtgat ttttgacagt 2100
   aaataacttt aggctttctc tcacaaattc cacaaagaca tggcattatt cgagtaaata 2160
   tgtcctttat atacagaaat ccgcctttac catccctaac acacttactc cccatactct 2220
   tacaaaaccc aatgaagcct gaggcaacag aagactgaaa tgcagatttg ttgattgact 2280
   ctgccaagat cttcttcacg ccttttgtga aatttcttga cagcctggac tgtattgtcc 2340
   ttatcaatgt tggcatctct tctttctcta acactcttcg acttgtcatg agtttggtcc 2400
   tcaagaccaa cctcaagtcc ccaaagctcg ctaaattgac ccatctgtag tctagagttt 2460
   gtctgatttc atcttcacta cacccggcat attgcaggaa tccggataaa gcctcatccc 2520
   ctcccctgct tatcaagttg ataaggtttt cctcaaagat tttgcctctc ttaatgtcat 2580
   tgaacacttt cctcgcgcag ttccttataa acattgtctc cttatcatca gaaaaaatag 2640
   cttcaatttt cctctgtaga cggtaccctc tagacccatc aacccagtct ttgacatctt 2700
   gttcttcaat agctccaaac ggagtctctc tgtatccaga gtatctaatc aattggttga 2760
   ctctaatgga aatctttgac actatatgag tgctaacccc attagcaata cattgatcac 2820
   aaattgtgtc tatggtctct gacagttgtg ttggagtttt acacttaacg ttgtgtagag 2880
   cagcagacac aaacttggtg agtaaaggag tctcttcacc catgacaaaa aatcttgact 2940
   taaactcagc aacaaaagtt cctatcacac tctttgggct gataaacttg tttaatttag 3000
   aagataagaa ttcatggaag cacaccattt ccagcagttc tgtcctgtct tgaaactttt 3060
   catcactaag gcaaggaatt tttataaggc taacctggtc atcgctggag gtataagtga 3120
   caggtatcac atcatacaat aagtcaagtg cataacacag aaattgttca gtaattagcc 3180
   catataaatc tgatgtgttg tgcaagattc cctggcccat gtccaagaca gacattatat 3240
   ggctggggac ctggtccctt gactgcagat actggtgaaa aaactcttca ccaacactag 3300
   tacagtcaca acccattaaa cctaaagatc tcttcaattt ccctacacag taggcttctg 3360
   caacattaat tggaacttca acgaccttat gaagatgcca tttgagaatg ttcattactg 3420
   gttcaagatt cacctttgtt ctatctctgg gattcttcaa ttctaatgtg tacaaaaaag 3480
   aaaggaaaag tgctgggctc atagttggtc cccatttgga gtggtcatat gaacaggaca 3540
   agtcaccatt gttaacagcc attttcatat cacagattgc acgttcgaat tccttttctg 3600
   aattcaagca tgtgtatttc attgaactac ccacagcttc tgagaagtct tcaactaacc 3660
   tggtcatcag cttagtgttg aggtctccca catacagttc tctatttgag ccaacctgct 3720
   ccttataact tagtccaaat ttcaagttcc ctgtatttga gctgatgctt gtgaactctg 3780
   taggagagtc gtctgaatag aaacataaat tccgtagggc tgcatttgta aaataacttt 3840
   tgtctagctt atcagcaatg gcttcagaat tgctttccct ggtactaagc cgaacctcat 3900
   cctttagtct cagaacttca ctggaaaagc ccaatctaga tctacttcta tgctcataac 3960
   tacccaattt ctgatcataa tgtccttgaa ttaaaagata cttgaagcat tcaaagaatt 4020
   catcttcttg gtaggctatt gttgtcaaat tttttaataa caaacccaaa gggcagatgt 4080
   cctgcggtgc ttcaagaaaa taagtcaatt taaatggaga tagataaaca gcatcacata 4140
   actctttata cacatcagac ctgagcacat ctggatcaaa atccttcacc tcatgcattg 4200
   acacctctgc tttaatctct ctcaacactc caaaaggggc ccacaatgac tcaagagact 4260
   ctcgctcatc aacagatgga tttttgattt tcaacttggt gatctcaact tttgtcccct 4320
   cactattagc catcttggct agtgtcattt gtacgtcatt tctaataccc tcaaaggccc 4380
   ttacttgatc ctctgttaaa ctctcataca tcactgataa ttcttcttga ttggttctgg 4440
   ttcttgaacc ggtgctcaca agacctgtta gattttttaa tattaagtag tccatggaat 4500
   caggatcaag attatacctg ccttttgttt taaacctctc agccatagta gaaacgcatg 4560
   ttgaaacaag tttctcctta tcataaacag aaagaatatt tccaagttcg tcgagcttgg 4620
   ggattaccac acttttattg cttgacagat ccagagctgt gctagtgatg ttaggcctgt 4680
   agggattgct tttcagttca cctgtaactt taagtcttcc tctattgaag agagaaatgc 4740
   agaaggacaa aatctcttta cacactcctg gaatttgagt atctgaggaa gtcttagcct 4800
   ctttggaaaa gaatctgtcc aatcctctta tcatggtgtc ctcttgttcc agtgttagac 4860
   tcccacttag aggggggttt acaacaacac aatcaaactt gactttgggc tcaataaact 4920
   tctcaaaaca cttgatttga tctgtcaggc gatcaggtgt ctctttggtt accaagtgac 4980
   acagataact aacatttaat agatatttaa accttcttgc aaagtaaaga tctgcatctt 5040
   ccccttcacc caaaattgtc tggaaaagtt ccacagccat cctctgaatc agcacctctg 5100
   atccagacat gcagtcgacc cttaactttg acatcaaatc cacatgatgg atttgatttg 5160
   catatgccat caagaaatat cttagacctt gtaaaaatgt ctggttcctt ttggaagggg 5220
   aacagagtac agctaacact aacaatctta atattggcct tgtcattgtc atgagttcgt 5280
   ggctaaaatc caaccagctg gtcatttcct cacacatttc aattaacaca tcctccgaaa 5340
   atataggcag gaaaaatctc tttggatcac agtaaaaaga gccttgttct tccaataccc 5400
   cattgatgga tagatagata gaatagcacc ttgacttctc acctgttttt tggtaaaaca 5460
   agagaccaaa tgtattcttt gtcagatgaa atctttgtac ataacactct cttagtctaa 5520
   cattcccaaa atatctagaa tactctcttt cattgattaa caatcgggag gaaaatgatg 5580
   tcttcatcga gttgaccaat gcaagggaaa tggaggacaa aatcctaaat aatttcttct 5640
   gctcaccttc cactaagctg ctgaatggct gatgtctaca gattttctca aattccttgt 5700
   taatagtata tctcatcact ggtctgtcag aaacaagtgc ctgagctaaa atcatcaagc 5760
   tatccatatc agggtgtttt attagttttt ccagctgtga ccagagatct tgatgagagt 5820
   tcttcaatgt tctggaacac gcttgaaccc acttggggct ggtcatcaat ttcttcctta 5880
   ttagtttaat cgcctccaga atatctagaa gtctgtcatt gactaacatt aacatttgtc 5940
   caacaactat tcccgcattt cttaacctta caattgcatc atcatgcgtt ttgaaaagat 6000
   cacaaagtaa attgagtaaa actaagtcca gaaacagtaa agtgtttctc ctggtgttga 6060
   aaacttttag acctttcact ttgttacaca cggaaagggc ttgaagataa cacctctcta 6120
   cagcatcaat agatatagaa ttctcatctg actggctttc catgttgact tcatctattg 6180
   gatgcaatgc gatagagtag actacatcca tcaacttgtt tgcacaaaaa gggcagctgg 6240
   gcacatcact gtctttgtgg cttcctaata agatcaagtc atttataagc ttagactttt 6300
   gtgaaaattt gaatttcccc aactgcttgt caaaaatctc cttcttaaac caaaacctta 6360
   actttatgag ttcttctctt atgacagatt ctctaatgtc tcctctaacc ccaacaaaga 6420
   gggattcatt taacctctca tcataaccca aagaattctt tttcaagcat tcgatgtttt 6480
   ctaatcccaa gctctggttt tttgtgttgg acaaactatg gatcaatcgc tggtattctt 6540
   gttcttcaat attaatctct tgcataaatt ttgatttctt taggatgtcg atcagcaacc 6600
   accgaactct ttcaacaacc caatcagcaa ggaatctatt gctgtagcta gatctgccat 6660
   caaccacagg aaccaacgta atccctgccc ttagtaggtc ggactttagg tttaagagct 6720
   ttgacatgtc actcttccat tttctctcaa actcatcagg attgacccta acaaaggttt 6780
   ccaataggat gagtgttttc cctgtgagtt tgaagccatc cggaatgact tttggaaggg 6840
   tgggacatag tatgccatag tcagacagga tcacatcaac aaacttctga tctgaattga 6900
   tctgacaggc gtgtgcctca caggactcaa gctctactaa acttgacaga agtttgaacc 6960
   cttccaacaa cagagagctg gggtgatgtt gagataaaaa gatgtccctt tggtatgcta 7020
   gctcctgtct ttctggaaaa tgctttctaa taaggctttt tatttcattt actgattcct 7080
   ccatgctcaa gtgccgccta ggatccactg tgcg 7114
<210> 5
<211> 3377
<212> RNA
<213> Lymphocytic choriomeningitis mammarenavirus Viruses (LCMV)
<220>
<223> Lymphocytic choriomeningitis virus clone 13 segment S, complete sequence.ACCESSION DQ361065-VERSION DQ361065.2-GI:116563461.-ssRNA viruses; ssRNA negative-strand viruses; Arenaviridae; Mammarenavirus.
<400> 5
   gcgcaccggg gatcctaggc tttttggatt gcgctttcct ctagatcaac tgggtgtcag 60
   gccctatcct acagaaggat gggtcagatt gtgacaatgt ttgaggctct gcctcacatc 120
   atcgatgagg tgatcaacat tgtcattatt gtgcttatcg tgatcacggg tatcaaggct 180
   gtctacaatt ttgccacctg tgggatattc gcattgatca gtttcctact tctggctggc 240
   aggtcctgtg gcatgtacgg tcttaaggga cccgacattt acaaaggagt ttaccaattt 300
   aagtcagtgg agtttgatat gtcacatctg aacctgacca tgcccaacgc atgttcagcc 360
   aacaactccc accattacat cagtatgggg acttctggac tagaattgac cttcaccaat 420
   gattccatca tcagtcacaa cttttgcaat ctgacctctg ccttcaacaa aaagaccttt 480
   gaccacacac tcatgagtat agtttcgagc ctacacctca gtatcagagg gaactccaac 540
   tataaggcag tatcctgcga cttcaacaat ggcataacca tccaatacaa cttgacattc 600
   tcagatgcac aaagtgctca gagccagtgt agaaccttca gaggtagagt cctagatatg 660
   tttagaactg ccttcggggg gaaatacatg aggagtggct ggggctggac aggctcagat 720
   ggcaagacca cctggtgtag ccagacgagt taccaatacc tgattataca aaatagaacc 780
   tgggaaaacc actgcacata tgcaggtcct tttgggatgt ccaggattct cctttcccaa 840
   gagaagacta agttcctcac taggagacta gcgggcacat tcacctggac tttgtcagac 900
   tcttcagggg tggagaatcc aggtggttat tgcctgacca aatggatgat tcttgctgca 960
   gagcttaagt gtttcgggaa cacagcagtt gcgaaatgca atgtaaatca tgatgaagaa 1020
   ttctgtgaca tgctgcgact aattgactac aacaaggctg ctttgagtaa gttcaaagag 1080
   gacgtagaat ctgccttgca cttattcaaa acaacagtga attctttgat ttcagatcaa 1140
   ctactgatga ggaaccactt gagagatctg atgggggtgc catattgcaa ttactcaaag 1200
   ttttggtacc tagaacatgc aaagaccggc gaaactagtg tccccaagtg ctggcttgtc 1260
   accaatggtt cttacttaaa tgagacccac ttcagtgacc aaatcgaaca ggaagccgat 1320
   aacatgatta cagagatgtt gaggaaggat tacataaaga ggcaggggag taccccccta 1380
   gcattgatgg accttctgat gttttccaca tctgcatatc tagtcagcat cttcctgcac 1440
   cttgtcaaaa taccaacaca caggcacata aaaggtggct catgtccaaa gccacaccga 1500
   ttaaccaaca aaggaatttg tagttgtggt gcatttaagg tgcctggtgt aaaaaccgtc 1560
   tggaaaagac gctgaagaac agcgcctccc tgactctcca cctcgaaaga ggtggagagt 1620
   cagggaggcc cagagggtct tagagtgtca caacatttgg gcctctaaaa attaggtcat 1680
   gtggcagaat gttgtgaaca gttttcagat ctgggagcct tgctttggag gcgctttcaa 1740
   aaatgatgca gtccatgagt gcacagtgcg gggtgatctc tttcttcttt ttgtccctta 1800
   ctattccagt atgcatctta cacaaccagc catatttgtc ccacactttg tcttcatact 1860
   ccctcgaagc ttccctggtc atttcaacat cgataagctt aatgtccttc ctattctgtg 1920
   agtccagaag ctttctgatg tcatcggagc cttgacagct tagaaccatc ccctgcggaa 1980
   gagcacctat aactgacgag gtcaacccgg gttgcgcatt gaagaggtcg gcaagatcca 2040
   tgccgtgtga gtacttggaa tcttgcttga attgtttttg atcaacgggt tccctgtaaa 2100
   agtgtatgaa ctgcccgttc tgtggttgga aaattgctat ttccactgga tcattaaatc 2160
   taccctcaat gtcaatccat gtaggagcgt tggggtcaat tcctcccatg aggtctttta 2220
   aaagcattgt ctggctgtag cttaagccca cctgaggtgg acctgctgct ccaggcgctg 2280
   gcctgggtga attgactgca ggtttctcgc ttgtgagatc aattgttgtg ttttcccatg 2340
   ctctccccac aatcgatgtt ctacaagcta tgtatggcca tccttcacct gaaaggcaaa 2400
   ctttatagag gatgttttca taagggttcc tgtccccaac ttggtctgaa acaaacatgt 2460
   tgagttttct cttggccccg agaactgcct tcaagaggtc ctcgctgttg cttggcttga 2520
   tcaaaattga ctctaacatg ttacccccat ccaacagggc tgcccctgcc ttcacggcag 2580
   caccaagact aaagttatag ccagaaatgt tgatgctgga ctgctgttca gtgatgaccc 2640
   ccagaactgg gtgcttgtct ttcagccttt caagatcatt aagatttgga tacttgactg 2700
   tgtaaagcaa gccaaggtct gtgagcgctt gtacaacgtc attgagcgga gtctgtgact 2760
   gtttggccat acaagccata gttagacttg gcattgtgcc aaattgattg ttcaaaagtg 2820
   atgagtcttt cacatcccaa actcttacca caccacttgc accctgctga ggctttctca 2880
   tcccaactat ctgtaggatc tgagatcttt ggtctagttg ctgtgttgtt aagttcccca 2940
   tatatacccc tgaagcctgg ggcctttcag acctcatgat cttggccttc agcttctcaa 3000
   ggtcagccgc aagagacatc agttcttctg cactgagcct ccccactttc aaaacattct 3060
   tctttgatgt tgactttaaa tccacaagag aatgtacagt ctggttgaga cttctgagtc 3120
   tctgtaggtc tttgtcatct ctcttttcct tcctcatgat cctctgaaca ttgctgacct 3180
   cagagaagtc caacccattc agaaggttgg ttgcatcctt aatgacagca gccttcacat 3240
   ctgatgtgaa gctctgcaat tctcttctca atgcttgcgt ccattggaag ctcttaactt 3300
   ccttagacaa ggacatcttg ttgctcaatg gtttctcaag acaaatgcgc aatcaaatgc 3360
   ctaggatcca ctgtgcg 3377
<210> 6
<211> 7229
<212> RNA
<213> Lymphocytic choriomeningitis mammarenavirus Viruses (LCMV)
<220>
<223> Lymphocytic choriomeningitis virus clone 13 segment L, complete sequence.-ACCESSION DQ361066-VERSION DQ361066.1-GI:86440167.-ssRNA viruses; ssRNA negative-strand viruses; Arenaviridae; Mammarenavirus
<400> 6
   gcgcaccggg gatcctaggc gtttagttgc gctgtttggt tgcacaactt tcttcgtgag 60
   gctgtcagaa gtggacctgg ctgatagcga tgggtcaagg caagtccaga gaggagaaag 120
   gcaccaatag tacaaacagg gccgaaatcc taccagatac cacctatctt ggccctttaa 180
   gctgcaaatc ttgctggcag aaatttgaca gcttggtaag atgccatgac cactaccttt 240
   gcaggcactg tttaaacctt ctgctgtcag tatccgacag gtgtcctctt tgtaaatatc 300
   cattaccaac cagattgaag atatcaacag ccccaagctc tccacctccc tacgaagagt 360
   aacaccgtcc ggccccggcc ccgacaaaca gcccagcaca agggaaccgc acgtcaccca 420
   acgcacacag acacagcacc caacacagaa cacgcacaca cacacacaca cacacccaca 480
   cgcacgcgcc cccaccaccg gggggcgccc ccccccgggg ggcggccccc cgggagcccg 540
   ggcggagccc cacggagatg cccatcagtc gatgtcctcg gccaccgacc cgcccagcca 600
   atcgtcgcag gacctcccct tgagtctaaa cctgcccccc actgtttcat acatcaaagt 660
   gctcctagat ttgctaaaac aaagtctgca atccttaaag gcgaaccagt ctggcaaaag 720
   cgacagtgga atcagcagaa tagatctgtc tatacatagt tcctggagga ttacacttat 780
   ctctgaaccc aacaaatgtt caccagttct gaatcgatgc aggaagaggt tcccaaggac 840
   atcactaatc ttttcatagc cctcaagtcc tgctagaaag actttcatgt ccttggtctc 900
   cagcttcaca atgatatttt ggacaaggtt tcttccttca aaaagggcac ccatctttac 960
   agtcagtggc acaggctccc actcaggtcc aactctctca aagtcaatag atctaatccc 1020
   atccagtatt cttttggagc ccaacaactc aagctcaaga gaatcaccaa gtatcaaggg 1080
   atcttccatg taatcctcaa actcttcaga tctgatatca aagacaccat cgttcacctt 1140
   gaagacagag tctgtcctca gtaagtggag gcattcatcc aacattcttc tatctatctc 1200
   acccttaaag aggtgagagc atgataaaag ttcagccaca cctggattct gtaattggca 1260
   cctaaccaag aatatcaatg aaaatttcct taaacagtca gtattattct gattgtgcgt 1320
   aaagtccact gaaattgaaa actccaatac cccttttgtg tagttgagca tgtagtccca 1380
   cagatccttt aaggatttaa atgcctttgg gtttgtcagg ccctgcctaa tcaacatggc 1440
   agcattacac acaacatctc ccattcggta agagaaccac ccaaaaccaa actgcaaatc 1500
   attcctaaac ataggcctct ccacattttt gttcaccacc tttgagacaa atgattgaaa 1560
   ggggcccagt gcctcagcac catcttcaga tggcatcatt tctttatgag ggaaccatga 1620
   aaaattgcct aatgtcctgg ttgttgcaac aaattctcga acaaatgatt caaaatacac 1680
   ctgttttaag aagttcttgc agacatccct cgtgctaaca acaaattcat caaccagact 1740
   ggagtcagat cgctgatgag aattggcaag gtcagaaaac agaacagtgt aatgttcatc 1800
   ccttttccac ttaacaacat gagaaatgag tgacaaggat tctgagttaa tatcaattaa 1860
   aacacagagg tcaaggaatt taattctggg actccacctc atgttttttg agctcatgtc 1920
   agacataaat ggaagaagct gatcctcaaa gatcttggga tatagccgcc tcacagattg 1980
   aatcacttgg ttcaaattca ctttgtcctc cagtagcctt gagctctcag gctttcttgc 2040
   tacataatca catgggttta agtgcttaag agttaggttc tcactgttat tcttcccttt 2100
   ggtcggttct gctaggaccc aaacacccaa ctcaaaagag ttgctcaatg aaatacaaat 2160
   gtagtcccaa agaagaggcc ttaaaaggca tatatgatca cggtgggctt ctggatgaga 2220
   ctgtttgtca caaatgtaca gcgttatacc atcccgattg caaactcttg tcacatgatc 2280
   atctgtggtt agatcctcaa gcagcttttt gatatacaga ttttccctat ttttgtttct 2340
   cacacacctg cttcctagag ttttgcaaag gcctataaag ccagatgaga tacaactctg 2400
   gaaagctgac ttgttgattg cttctgacag cagcttctgt gcaccccttg tgaatttact 2460
   acaaagtttg ttctggagtg tcttgatcaa tgatgggatt ctttcctctt ggaaagtcat 2520
   cactgatgga taaaccacct tttgtcttaa aaccatcctt aatgggaaca tttcattcaa 2580
   attcaaccag ttaacatctg ctaactgatt cagatcttct tcaagaccga ggaggtctcc 2640
   caattgaaga atggcctcct ttttatctct gttaaatagg tctaagaaaa attcttcatt 2700
   aaattcacca tttttgagct tatgatgcag tttccttaca agctttctta caacctttgt 2760
   ttcattagga cacagttcct caatgagtct ttgtattctg taacctctag aaccatccag 2820
   ccaatctttc acatcagtgt tggtattcag tagaaatgga tccaaaggga aattggcata 2880
   ctttaggagg tccagtgttc tcctttggat actattaact agggagactg ggacgccatt 2940
   tgcgatggct tgatctgcaa ttgtatctat tgtttcacaa agttgatgtg gctctttaca 3000
   cttgacattg tgtagcgctg cagatacaaa ctttgtgaga agagggactt cctcccccca 3060
   tacatagaat ctagatttaa attctgcagc gaacctccca gccacacttt ttgggctgat 3120
   aaatttgttt aacaagccgc tcagatgaga ttggaattcc aacaggacaa ggacttcctc 3180
   cggatcactt acaaccaggt cactcagcct cctatcaaat aaagtgatct gatcatcact 3240
   tgatgtgtaa gcctctggtc tttcgccaaa gataacacca atgcagtagt tgatgaacct 3300
   ctcgctaagc aaaccataga agtcagaagc attatgcaag attccctgcc ccatatcaat 3360
   aaggctggat atatgggatg gcactatccc catttcaaaa tattgtctga aaattctctc 3420
   agtaacagtt gtttctgaac ccctgagaag ttttagcttc gacttgacat atgatttcat 3480
   cattgcattc acaacaggaa aggggacctc gacaagctta tgcatgtgcc aagttaacaa 3540
   agtgctaaca tgatctttcc cggaacgcac atactggtca tcacctagtt tgagattttg 3600
   tagaaacatt aagaacaaaa atgggcacat cattggtccc catttgctgt gatccatact 3660
   atagtttaag aacccttccc gcacattgat agtcattgac aagattgcat tttcaaattc 3720
   cttatcattg tttaaacagg agcctgaaaa gaaacttgaa aaagactcaa aataatcttc 3780
   tattaacctt gtgaacattt ttgtcctcaa atctccaata tagagttctc tatttccccc 3840
   aacctgctct ttataagata gtgcaaattt cagccttcca gagtcaggac ctactgaggt 3900
   gtatgatgtt ggtgattctt ctgagtagaa gcacagattt ttcaaagcag cactcataca 3960
   ttgtgtcaac gacagagctt tactaaggga ctcagaatta ctttccctct cactgattct 4020
   cacgtcttct tccagtttgt cccagtcaaa tttgaaattc aagccttgcc tttgcatatg 4080
   cctgtatttc cctgagtacg catttgcatt catttgcaac agaatcatct tcatgcaaga 4140
   aaaccaatca ttctcagaaa agaactttct acaaaggttt tttgccatct catcgaggcc 4200
   acactgatct ttaatgactg aggtgaaata caaaggtgac agctctgtgg aaccctcaac 4260
   agcctcacag ataaatttca tgtcatcatt ggttagacat gatgggtcaa agtcttctac 4320
   taaatggaaa gatatttctg acaagataac ttttcttaag tgagccatct tccctgttag 4380
   aataagctgt aaatgatgta gtccttttgt atttgtaagt ttttctccat ctcctttgtc 4440
   attggccctc ctacctcttc tgtaccgtgc tattgtggtg ttgacctttt cttcgagact 4500
   tttgaagaag cttgtctctt cttctccatc aaaacatatt tctgccaggt tgtcttccga 4560
   tctccctgtc tcttctccct tggaaccgat gaccaatcta gagactaact tggaaacttt 4620
   atattcatag tctgagtggc tcaacttata cttttgtttt cttacgaaac tctccgtaat 4680
   ttgactcaca gcactaacaa gcaatttgtt aaagtcatat tccagaagtc gttctccatt 4740
   tagatgctta ttaaccacca cacttttgtt actagcaaga tctaatgctg tcgcacatcc 4800
   agagttagtc atgggatcta ggctgtttag cttcttctct cctttgaaaa ttaaagtgcc 4860
   gttgttaaat gaagacacca ttaggctaaa ggcttccaga ttaacacctg gagttgtatg 4920
   ctgacagtca atttctttac tagtgaatct cttcatttgc tcatagaaca cacattcttc 4980
   ctcaggagtg attgcttcct tggggttgac aaaaaaacca aattgacttt tgggctcaaa 5040
   gaacttttca aaacatttta tctgatctgt tagcctgtca ggggtctcct ttgtgatcaa 5100
   atgacacagg tatgacacat tcaacataaa tttaaatttt gcactcaaca acaccttctc 5160
   accagtacca aaaatagttt ttattaggaa tctaagcagc ttatacacca ccttctcagc 5220
   aggtgtgatc agatcctccc tcaacttatc cattaatgat gtagatgaaa aatctgacac 5280
   tattgccatc accaaatatc tgacactctg tacctgcttt tgatttctct ttgttgggtt 5340
   ggtgagcatt agcaacaata gggtcctcag tgcaacctca atgtcggtga gacagtcttt 5400
   caaatcagga catgatctaa tccatgaaat catgatgtct atcatattgt ataagacctc 5460
   atctgaaaaa attggtaaaa agaacctttt aggatctgca tagaaggaaa ttaaatgacc 5520
   atccgggcct tgtatggagt agcaccttga agattctcca gtcttctggt ataataggtg 5580
   gtattcttca gagtccagtt ttattacttg gcaaaacact tctttgcatt ctaccacttg 5640
   atatctcaca gaccctattt gattttgcct tagtctagca actgagctag ttttcatact 5700
   gtttgttaag gccagacaaa cagatgataa tcttctcagg ctctgtatgt tcttcagctg 5760
   ctctgtgctg ggttggaaat tgtaatcttc aaacttcgta taatacatta tcgggtgagc 5820
   tccaattttc ataaagttct caaattcagt gaatggtatg tggcattctt gctcaaggtg 5880
   ttcagacagt ccgtaatgct cgaaactcag tcccaccact aacaggcatt tttgaatttt 5940
   tgcaatgaac tcactaatag atgccctaaa caattcctca aaagacacct ttctaaacac 6000
   ctttgacttt tttctattcc tcaaaagtct aatgaactcc tctttagtgc tgtgaaagct 6060
   taccagccta tcattcacac tactatagca acaacccacc cagtgtttat cattttttaa 6120
   ccctttgaat ttcgactgtt ttatcaatga ggaaagacac aaaacatcca gatttaacaa 6180
   ctgtctcctt ctagtattca acagtttcaa actcttgact ttgtttaaca tagagaggag 6240
   cctctcatat tcagtgctag tctcacttcc cctttcgtgc ccatgggtct ctgcagttat 6300
   gaatctcatc aaaggacagg attcgactgc ctccctgctt aatgttaaga tatcatcact 6360
   atcagcaagg ttttcataga gctcagagaa ttccttgatc aagccttcag ggtttacttt 6420
   ctgaaagttt ctctttaatt tcccactttc taaatctctt ctaaacctgc tgaaaagaga 6480
   gtttattcca aaaaccacat catcacagct catgttgggg ttgatgcctt cgtggcacat 6540
   cctcataatt tcatcattgt gagttgacct cgcatctttc agaattttca tagagtccat 6600
   accggagcgc ttgtcgatag tagtcttcag ggactcacag agtctaaaat attcagactc 6660
   ttcaaagact ttctcatttt ggttagaata ctccaaaagt ttgaataaaa ggtctctaaa 6720
   tttgaagttt gcccactctg gcataaaact attatcataa tcacaacgac catctactat 6780
   tggaactaat gtgacacccg caacagcaag gtcttccctg atgcatgcca atttgttagt 6840
   gtcctctata aatttcttct caaaactggc tggagtgctc ctaacaaaac actcaagaag 6900
   aatgagagaa ttgtctatca gcttgtaacc atcaggaatg ataagtggta gtcctgggca 6960
   tacaattcca gactccacca aaattgtttc cacagactta tcgtcgtggt tgtgtgtgca 7020
   gccactcttg tctgcactgt ctatttcaat gcagcgtgac agcaacttga gtccctcaat 7080
   cagaaccatt ctgggttccc tttgtcccag aaagttgagt ttctgccttg acaacctctc 7140
   atcctgttct atatagttta aacataactc tctcaattct gagatgattt catccattgc 7200
   gcatcaaaaa gcctaggatc ctcggtgcg 7229

## Beschreibung

### ANWENDUNGSGEBIET UND STAND DER TECHNIK

Die Erfindung betrifft Arenaviren zur Anwendung bei der Behandlung, von Tumoren wie in den Ansprüchen definiert.

Die Arenaviren gehören zur Familie der humanpathogenen, pleomorphen RNA-Viren. Erkrankungen mit diesen Viren gehören aufgrund ihres natürlichen Reservoirs in Tieren, überwiegend Nagern, zu den Zoonosen. Als Zoonosen bezeichnet man Erkrankungen, die vom Tier auf den Menschen und umgekehrt vom Menschen auf das Tier übertragbar sind.

Wenigstens acht Arenaviren sind dafür bekannt, dass sie im Menschen eine Krankheit verursachen. Typisch sind aseptische Meningitis und hämorrhagisches Fieber. Bekannte Viren, welche im Menschen eine Erkrankung auslösen können, stellen das Lymphozytäre-Choriomeningitis-Virus (LCMV), Guanarito-Virus (GTOV), Junin-Virus (JUNV), Lassa-Virus (LASV), Lujo-Virus (LUJV), Machupo-Virus (MACV), Sabia-Virus (SABV) sowie das Whitewater-Arroyo-Virus (WWAV) dar.

Arenaviren werden allgemein in zwei Gruppen, nämlich in die Altwelt-Arenaviren und die Neuwelt-Arenaviren, unterteilt. Diese Gruppen unterscheiden sich geografisch und genetisch. Altwelt-Arenaviren, wie beispielsweise das Lymphozytäre-Choriomeningitis-Virus, wurden in Ländern der östlichen Hemisphäre, wie beispielsweise europäischen, asiatischen und afrikanischen Ländern, gefunden. Dagegen wurden Neuwelt-Arenaviren in Ländern der westlichen Hemisphäre, wie beispielsweise Argentinien, Bolivien, Venezuela, Brasilien und den Vereinigten Staaten von Amerika, gefunden.

Der Name der Virenfamilie leitet sich vom Lateinischen arenosus (sandig) bzw. arena (Sand) her, um damit die sandige ribosomale Struktur im Inneren der Virionen zu beschreiben. Die Virionen der Arenaviren haben eine runde bis unregelmäßige Gestalt und sind je nach Spezies und Präparation des Untersuchungsmaterials 50 nm bis 300 nm, meistens zwischen 110 nm und 130 nm, im Durchmesser groß. In die Virushülle sind 8 nm bis 10 nm lange, keulenförmige Glykoprotein-Spikes eingelagert. Die einzelnen Spikes bestehen aus einem Tetramer des viralen Hüllproteins.

Die Virionen enthalten weiterhin zwei ringförmig geschlossene und eine helikale Symmetrie aufweisende Kapside. Die Länge der Kapside variiert zwischen 450 nm und 1300 nm. Jeweils ein Molekül der viralen RNA(ribonucleic acid)-Polymerase (L-Protein) ist an sie angelagert.

Jedes Kapsid enthält ein Molekül einer einzelsträngigen RNA mit gemischter (d.h. ambisense, +/-) Polarität. Die beiden einzelsträngigen RNA-Moleküle stellen dabei das virale Genom dar. Sie werden als L (large) und S (small) bezeichnet und sind etwa 7,5 kb (Kilobasen) bzw. 3.5 kb (Kilobasen) groß. Obwohl die Kapside ringförmig geschlossen sind, sind die RNA-Stränge linear und damit nicht zirkulär. Eine 19 bis 30 Basen lange Sequenz am 3'-Ende der RNA ist an beiden Strängen vorhanden und auch innerhalb der Virusfamilie konserviert.

Morphologisch sehr außergewöhnlich ist die Anwesenheit einer wechselnden Anzahl von zellulären Ribosomen innerhalb der Virionen, welche den Viruspartikeln ihr "sandiges" Aussehen verleihen. Ebenso findet man in gereinigten Viruspräparationen eine Anzahl unterschiedlicher zellulärer RNAs (darunter auch ribosomale RNA) sowie replikative Formen der viralen RNA, so verschiedene virale mRNAs (messenger ribonucleic acids, an die Ribosomen gebunden) und vollständige komplementäre Stränge des Virusgenoms. Diese nicht-genomischen RNAs befinden sich in wechselnder Menge alle außerhalb der oben genannten Kapside.

Die Verwendung von Arenaviren als Impfvektoren ist bekannt. Ein prominentes Beispiel ist der gegen argentinisches hämorrhagisches Fieber eingesetzte Impfvirus Candid #1. Hierbei handelt es sich um eine Impfvariante des Junin-Virus.

Aus der WO 2009/083210 A1 ist die Verwendung von replikationsdefekten, d.h. gentechnologisch modifizierten, Arenavirus-Partikel (Virionen) unter anderem zur Behandlung von neoplastischen Erkrankungen, wie beispielsweise Melanom, Prostatakarzinom, Mammakarzinom und Lungenkarzinom, bekannt. In der Publikation "Development of replication-defective lymphocytic choriomeningitis virus vectors for the induction of potent CD8+ T cell immunity" (Nature Medicine, Bd. 16, Nr. 3, März 2010, S. 339-345; doi: 10.1038/nm.2104) wird als potentielles Anwendungsgebiet für solche Viruspartikel die Krebsimmuntherapie erwähnt.

Ferner ist aus der WO 2006/008074 A1 die Verwendung von Verpackungszellen, welche retrovirale, mit Arenavirus-Glykoprotein pseudotypisierte Virionen produzieren, zur Gentherapie solider Tumore bekannt.

Kelly, E. & Russell, S. (2007) Molecular Therapy, Bd. 15, Nr. 4, 651 - 659 beschreibt die Geschichte der onkolytischen Viren: Von der Entstehung bis zur Gentechnik. Molomut, N. & Padnos, M. (1965) Nature, Bd. 208, Nr. 5014, 948 - 950 offenbart die Inhibition von transplantierbaren und spontanen Mäusetumoren durch das M-P-Virus. Reiserova, L. et al. (1999) Virology, Bd. 257, Nr. 1, 73 - 83 offenbart die Identifizierung des MaTu-MX-Erregers als neuer Stamm des lymphozytischen Choriomeningitis-Virus (LCMV) und serologische Hinweise auf eine horizontale Ausbreitung von LCMV in der menschlichen Bevölkerung. Koma, T et al. (2013) J. Mol. Biol., Bd. 425, Nr. 24 , 4893 - 4903 beschreibt die angeborene Immunantwort auf Arenavirus-Infektionen mit Fokus auf die hochpathogenen hämorrhagischen Neuwelt-Arenaviren. Groseth, A. et al. (2011) PLOS Neglected tropical diseases, Bd. 5, Nr. 5, e1137 offenbart, dass Infektion mit dem Tacaribe-Virus, nicht aber mit dem Junin-Virus, die Freisetzung von Zytokinen aus primären menschlichen Monozyten und Makrophagen induziert. Webb, H. E. et al. (1975) Clinical Oncology 1975, Bd. 1, Nr. 2, 157 - 169 offenbart die Behandlung von 18 Fällen bösartiger Erkrankungen mit einem Arenavirus. Schadler, Keri L. et al. (2014) Cancer research, Bd. 74, Nr. 8, 2171 - 2181 offenbart Immunosurveillance durch Antiangiogenese, insbesondere Tumorwachstumsstopp durch T-Zell-erzeugtes Thrombospondin-1.

Die im Stand der Technik beschriebenen Methoden zur Behandlung von Tumoren beruhen auf der Verwendung von gentechnologisch sehr aufwändig herzustellenden Viruspartikeln. Bei gentherapeutischen Behandlungsmethoden kommt hinzu, dass eine ausreichende, therapeutisch wirksame Transduktion des Tumorgewebes mit gentechnisch hergestellten Virionen oder Verpackungszellen, welche Virionen produzieren, häufig nicht erzielbar ist.

### AUFGABE UND LÖSUNG

Der vorliegenden Erfindung liegt daher die Aufgabe zugrunde, eine gegenüber dem Stand der Technik einfachere und insbesondere effizientere Therapielösung für Tumore, insbesondere Karzinome und Sarkome, bereitzustellen.

Diese Aufgabe wird erfindungsgemäß gelöst durch ein Arenavirus zur Anwendug gemäß unabhängigem Anspruch 1. Bevorzugte Ausführungsformen sind in den abhängigen Ansprüchen definiert.

Gemäß einem ersten Aspekt betrifft die Erfindung ein Arenavirus zur Anwendung bei der Behandlung eines Tumors, vorzugsweise eines malignen, d.h. bösartigen, Tumors beim Menschen, wobei eine Tumorregression bewirkt wird, wobei das Arenavirus frei von genomischer Fremd-RNA ist, wobei das Arenavirus Lymphozytäre-Choriomeningitis-Virus, WE-Stamm, ist.

Das Arenavirus zeichnet sich dadurch aus, dass es frei von genomischer Fremd-RNA ist, d.h. keine genomische Fremd-RNA aufweist. Mit anderen Worten ist das Genom (Erbgut) des Arenavirus frei von Fremd-RNA bzw. weist vorzugsweise keine Fremd-RNA auf.

Unter dem Ausdruck "genomische Fremd-RNA" soll im Sinne der vorliegenden Erfindung eine RNA (ribonucleic acid, Ribonukleinsäure) bzw. RNA-Sequenz verstanden werden, welche im Genom eines Wildtyp-Arenavirus oder im Genom einer Mutante eines Wildtyp-Arenavirus (mutiertes Arenavirus), insbesondere im Genom einer natürlichen Mutante eines Wildtyp-Arenavirus (natürlich mutiertes Arenavirus), nicht vorkommt bzw. nicht enthalten ist. Beispiele für Fremd-RNA stellen artifizielle bzw. synthetische RNA-Moleküle, RNA von Organismen sowie RNA von anderen Viren dar.

Unter dem Ausdruck "Wildtyp-Arenavirus" soll im Sinne der vorliegenden Erfindung (in Übereinstimmung mit dem fachmännischen Verständnis) ein Arenavirus verstanden werden, dessen Genom die in der Natur auftretende genetische Normalform darstellt.

Unter dem Ausdruck "Mutante eines Wildtyp-Arenavirus" bzw. "mutiertes Arenavirus" soll im Sinne der vorliegenden Erfindung (in Übereinstimmung mit dem fachmännischen Verständnis) ein Arenavirus verstanden werden, dessen Genom gegenüber dem Wildtyp-Genom eine spontane, d.h. natürlich verursachte, oder durch Mutagene induzierte Veränderung aufweist.

Dementsprechend soll unter dem Ausdruck "natürliche Mutante eines Wildtyp-Arenavirus" bzw. "natürlich mutiertes Arenavirus" im Sinne der vorliegenden Erfindung (in Übereinstimmung mit dem fachmännischen Verständnis) ein Arenavirus verstanden werden, dessen Genom gegenüber dem Wildtyp-Genom eine spontane, d.h. natürlich verursachte, Veränderung aufweist. Ein natürlich mutiertes Arenavirus lässt sich vorzugsweise durch Passage, insbesondere serielle Passage, herstellen, worauf im Folgenden noch näher eingegangen werden wird.

Die Erfindung beruht auf dem überraschenden Befund, dass Arenaviren ohne genomische Fremd-RNA in der Lage sind, eine Tumorregression zu bewirken. Die Tumorregression beruht auf einer durch die Arenaviren verursachten Aktivierung bzw. Stimulierung von angeborenen und adaptiven Immunzellen. Die aktivierten Immunzellen sezernieren vermehrt antitumorale Zytokine wie Interferon-α und Interferon-γ, wodurch der Tumor bekämpft bzw. abgestoßen wird. Ein weiterer überraschender Befund stellt die Erkenntnis dar, dass die Arenaviren im Falle einer Tumormanifestation eine signifikant erhöhte Sezernierung von antitumoralen Zytokinen bewirken. Arenaviren ohne genomische Fremd-RNA eignen sich somit zur Anwendung bei der Tumorbehandlung. Dies wurde vom Anmelder erfolgreich anhand von Tierexperimenten verifiziert. Hierzu wurden unter anderem Mäuse eingesetzt, in welchen ein Wachstum humaner Tumoren möglich ist.

Das Arenavirus ist frei von nichtgenomischer Fremd-RNA. Unter dem Ausdruck "nichtgenomische Fremd-RNA" soll im Sinne der vorliegenden Erfindung eine RNA bzw. RNA-Sequenz außerhalb des Arenavirusgenoms verstanden werden, welche in einem Wildtyp-Arenavirus oder einer Mutante eines Wildtyp-Arenavirus (mutiertes Arenavirus), insbesondere natürlichen Mutante eines Wildtyp-Arenavirus (natürlich mutiertes Arenavirus), nicht vorkommt bzw. nicht enthalten ist. Mit anderen Worten weist das Arenavirus bei dieser Ausführungsform insgesamt keine Fremd-RNA, d.h weder genomische Fremd-RNA noch nichtgenomische Fremd-RNA, auf.

Unter dem Ausdruck "Passage" soll im Sinne der vorliegenden Erfindung in Übereinstimmung mit dem fachmännischen Verständnis ein vielfaches, regelmäßiges Einbringen des Arenavirus in Wirtstiere und/oder Wirtszellen verstanden werden. Unter dem Ausdruck "serielle Passage" soll im Sinne der vorliegenden Erfindung in Übereinstimmung mit dem fachmännischen Verständnis ein vielfaches, regelmäßiges Einbringen des Arenavirus in unterschiedliche Wirtstiere, vorzugsweise derselben Art, und/oder unterschiedliche Zellen, vorzugsweise desselben Typs, verstanden werden. Durch den vielfachen Wechsel der Umgebung (Wirtstier und/oder Wirtszelle) ist das Arenavirus einem erhöhten Adaptionszwang bzw. Mutationsdruck ausgesetzt, wodurch sich die Wahrscheinlichkeit erhöht, dass es zu unter Tumorregressionsgesichtspunkten vorteilhaften Mutationen im Genom des Arenavirus kommt.

In einer Ausführungsform ist der Tumor ausgewählt aus der Gruppe aufweisend oder bestehend aus Karzinom, Melanom, Blastom, Lymphom und Sarkom.

Unter dem Ausdruck "Karzinom" soll im Sinne der vorliegenden Erfindung (in Übereinstimmung mit dem fachmännischen Verständnis) eine maligne Neoplasie epithelialen Ursprungs verstanden werden.

Unter dem Ausdruck "Sarkom" soll im Sinne der vorliegenden Erfindung (in Übereinstimmung mit dem fachmännischen Verständnis) eine maligne Neoplasie mesodermalen Ursprungs verstanden werden.

Unter dem Ausdruck "Melanom" soll im Sinne der vorliegenden Erfindung (in Übereinstimmung mit dem fachmännischen Verständnis) eine maligne Neoplasie melanozytären Ursprungs verstanden werden.

Unter dem Ausdruck "Lymphom" soll im Sinne der vorliegenden Erfindung (in Übereinstimmung mit dem fachmännischen Verständnis) eine maligne Neoplasie lymphozytären Ursprungs verstanden werden.

Unter dem Ausdruck "Blastom" soll im Sinne der vorliegenden Erfindung (in Übereinstimmung mit dem fachmännischen Verständnis) eine maligne Neoplasie embryonalen Ursprungs verstanden werden.

Das Karzinom ist in einer bevorzugten Ausführungsform ausgewählt aus der Gruppe aufweisend oder bestehend aus Analkarzinom, Bronchialkarzinom, Lungenkarzinom, Endometriumkarzinom, Gallenblasenkarzinom, hepatozelluläres Karzinom, Hodenkarzinom, kolorektales Karzinom, Larynxkarzinom, Speiseröhrenkrebs, Magenkarzinom, Mammakarzinom, Nierenkarzinom, Ovarialkarzinom, Pankreastumor, Pharynxkarzinom, Prostatakarzinom, Schilddrüsenkarzinom und Cervixkarzinom.

Das Sarkom ist in einer bevorzugten Ausführungsform ausgewählt aus der Gruppe aufweisend oder bestehend aus Angiosarkom, Chondrosarkom, Ewing-Sarkom, Fibrosarkom, Kaposi-Sarkom, Liposarkom, Leiomyosarkom, Malignes Fibröses Histiozytom, neurogenes Sarkom, Osteosarkom und Rhabdomyosarkom.

Bei dem Arenavirus handelt es sich um das Lymphozytäre-Choriomeningitis-Virus (LCMV), WE-Stamm.

Das Arenavirus kann für eine Verabreichung in Form von Virionen vor, d.h. in Form von Arenaviruspartikeln, welche sich außerhalb einer Zelle befinden, vorliegen.

Alternativ ist das Arenavirus für eine lokale, insbesondere intramuskuläre, intraperitoneale oder subkutane, Verabreichung vorgesehen, vorzugsweise hergerichtet. Mit anderen Worten wird das Arenavirus für eine lokale, insbesondere intramuskuläre, intraperitoneale oder subkutane, Verabreichung verwendet.

Bevorzugt ist das Arenavirus zur lokalen Verabreichung in einer Dosis von 1 PFU (Plaque Forming Unit)/kg Körpergewicht bis 10¹² PFU/kg Körpergewicht, insbesondere 10² PFU/kg Körpergewicht bis 10⁶ PFU/kg Körpergewicht, bevorzugt 10³ PFU/kg Körpergewicht bis 10⁵ PFU/kg Körpergewicht, vorgesehen, vorzugsweise hergerichtet. Mit anderen Worten wird das Arenavirus bevorzugt zur lokalen Verabreichung in einer Dosis von 1 PFU (Plaque Forming Unit)/kg Körpergewicht bis 10¹² PFU/kg Körpergewicht, insbesondere 10² PFU/kg Körpergewicht bis 10⁶ PFU/kg Körpergewicht, bevorzugt 10³ PFU/kg Körpergewicht bis 10⁵ PFU/kg Körpergewicht, verwendet.

Alternativ ist das Arenavirus für eine systemische, insbesondere intravenöse, Verabreichung vorgesehen, vorzugsweise hergerichtet. Mit anderen Worten wird das Arenavirus in einer alternativen Ausführungsform für eine systemische, insbesondere intravenöse, Verabreichung verwendet.

Vorzugsweise ist das Arenavirus zur systemischen Verabreichung in einer Dosis von 1 PFU/kg Körpergewicht bis 10¹² PFU/kg Körpergewicht, insbesondere 10² PFU/kg Körpergewicht bis 10⁶ PFU/kg Körpergewicht, bevorzugt 10³ PFU/kg Körpergewicht bis 10⁵ PFU/kg Körpergewicht, vorgesehen, vorzugsweise hergerichtet. Mit anderen Worten wird das Arenavirus vorzugsweise zur systemischen Verabreichung in einer Dosis von 1 PFU/kg Körpergewicht bis 10¹² PFU/kg Körpergewicht, insbesondere 10² PFU/kg Körpergewicht bis 10⁶ PFU/kg Körpergewicht, bevorzugt 10³ PFU/kg Körpergewicht bis 10⁵ PFU/kg Körpergewicht, verwendet.

Gemäß einem zweiten Aspekt betrifft die Erfindung ein Medikament zur Anwendung bei der Behandlung eines Tumors beim Menschen, insbesondere malignen Tumors.

Das Medikament zeichnet sich dadurch aus, dass es ein Arenavirus wie oben ausgeführt aufweist.

Das Medikament weist vorzugsweise ferner einen pharmazeutisch verträglichen Träger auf. Der Träger kann insbesondere ausgewählt sein aus der Gruppe aufweisend oder bestehend aus Wasser, Salzlösung, Pufferlösung und Zellkulturmedium.

Das Medikament weist ferner einen Wirkstoff auf. Der Wirkstoff kann insbesondere ein Zytostatikum, ein Antikörper und/oder ein Zytokin sein.

Bezüglich weiterer Merkmale und Vorteile des Medikaments, insbesondere des Arenavirus sowie des Tumors, wird zur Vermeidung von Wiederholungen vollständig auf die bisherige Beschreibung Bezug genommen.

Weitere Merkmale und Vorteile der Erfindung ergeben sich aus der nachfolgenden Beschreibung bevorzugter Ausführungsformen in Form von Ausführungsbeispielen, den dazugehörigen Figuren sowie den Ansprüchen.

### Beispielteil

### 1. Methoden und Materialien

### 1. 1 Mäuse

Wenn nicht anderes erwähnt, waren die verwendeten Mäuse auf einem C57BL/6 Hintergrund. *Map3k14*^{*aly*/*aly*} Mäusen fehlt NF-kB Signale und sind somit stark immunsupprimiert. *Irf3 × Ir7*^{*-*/*-*}Mäuse können kein Interferon produzieren. NOD.SCID Mäuse haben kein adaptives Immunsystem. Somit ist ein Wachstum humaner Tumoren in diesen Mäusen möglich. LoxP-Tag Mäuse entwickeln spontan Lebertumoren.

### 1.2 Zelllinien und Reagenzien

MOPC Zellen sind murine Oropharynx Karzinom Zellen. Mc38 sind murine Kolonzarzinomzellen. Raw Zellen sind immortalisierte Makrophagen. A431 sind humane Lungenkarzinomzellen; Sw40 sind humane Kolonkarzinomzellen, Hela sind humane Cervixkarzinomzellen. Primäre Makrophagen wurden mittels M-CSF aus Knochenmarksvorläuferzellen gezüchtet. Zellen wurden in Dulbeccos modifiziertem Eagle-Medium mit 10% fötalem Rinderserum (Sigma-Aldrich), 2 mmol / I L-Glutamin und 100 U / ml Penicillin gehalten. Alle Zellen wurden in 5% CO2 kultiviert.

### 1.3 Viren

Die LCMV-Stamm WE wurde aus dem Labor von Prof. Zinkernagel (Experimentelle Immunologie, Zürich, Schweiz) erhalten und wurde in L929-Zellen vermehrt. Candid#1 wurde von Professor Paula Cannon (University of Southern California) erhalten.

### 1.4 Tumorwachstum und Behandlungen

Ungefähr 5 × 10⁵ Tumorzellen (in 100microL) wurden subkutan in die rechte Flanke in 6 bis 12 Wochen alten Mäusen injiziert. Der längste Tumordurchmesser wurde durch gemessen. Mäuse wurden mit peritumoralen Injektionen von 2 × 10⁴ PFU LCMV-WE oder Candid#1 behandelt (in 100-200microL).

### 1.5 Morphometrische Analyse von Tumorgefäßen

Morphometrische Analysen wurden mit aufeinanderfolgenden Gefrierschnitten durchgeführt, bei welchen der endotheliale Zellmarker CD31 angefärbt wurde. Die Quantifizierung der Gefäßdichte (MVD) wurde unter Verwendung des Durchschnitts von drei Tumorschnitten berechnet. MVD wurde als Zahl der Gefäße je Tumorfläche berechnet.

### 1.6 Der Nachweis von Hypoxie

Hypoxische Tumorbereiche wurden durch die Bildung von pimonidazole Addukte nach Injektion von Pimonidazol in Tumor-transplantierte Tiere für 30 min detektiert. Die Tumorschnitte wurden unter Verwendung des Hypoxyprobe-1 Plus-Kits nach Vorschrift des Herstellers (Pharmacia Natur International, Inc.) gefärbt.

### 1.7 IFN-α ELISA

Serum IFN-α-Spiegel wurden durch ELISA nach den Herstellerangaben (Research Diagnostics RDI, Flanders, NJ) bestimmt.

### 1.8 Statistische Analyse

Die Mittelwerte wurden unter Verwendung eines ungepaarten Student zweiseitigen t-Test verglichen. Die Daten werden als Mittelwert ± SEM dargestellt. Das Niveau der statistischen Signifikanz wurde bei p <0,05 festgelegt.

### 2. Untersuchungen

**2.1** Immortalisierte Makrophagen (Tumorzellen) und aus primärem Knochenmark generierte Makrophagen (primär) wurden mit LCMV (Stamm WE) infiziert. Die Replikation wurde nach 24 Stunden gemessen (n = 3).

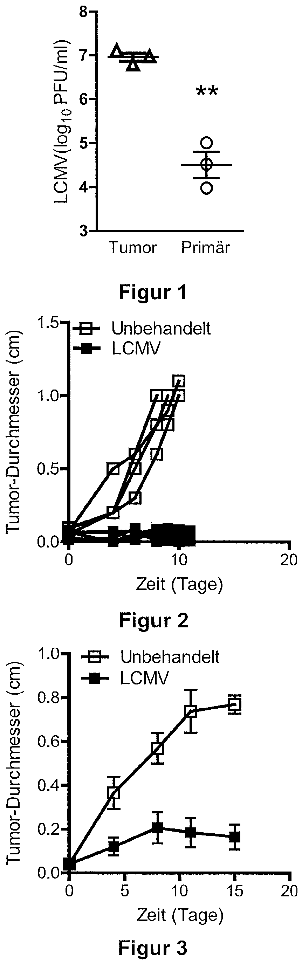

Dabei konnte nachgewiesen werden, dass sich LCMV (Stamm WE) sowohl in immortalisierten als auch gesunden Zellen repliziert. Die erhaltenen Ergebnisse sind in Figur 1 grafisch dargestellt.

Die Figur 1 hat die folgende Legende:
Ordinate: LCMV (log₁₀ PFU/ml)
Abszisse: Tumorzellen/gesunde Makrophagen (primär)

**2.2** WT C57BL/6-Mäuse wurden mit 5 × 10⁵ MOPC Zellen (Tag 3) behandelt. Eine Gruppe von Mäusen wurde zusätzlich mit 2 × 10⁴ PFU LCMV (Stamm WE) peritumoral behandelt (Tag 0). Das Tumorwachstum wurde beobachtet.

Dabei konnte nachgewiesen werden, dass die Behandlung mit LCMV eine fast vollständige Tumorregression bewirkte. Die erhaltenen Ergebnisse sind in Figur 2 grafisch dargestellt.

Die Figur 2 hat die folgende Legende:
Ordinate: Tumor-Durchmesser (cm)
Abszisse: Zeit (Tage)

**2.3** WT C57BL/6-Mäuse wurden mit 5 × 10⁵ MC38 Zellen (Tag 3) behandelt. Eine Gruppe von Mäusen wurde zusätzlich mit 2 × 10⁴ PFU LCMV (Stamm WE) peritumoral behandelt (Tag 0). Das Tumorwachstum wurde beobachtet.

Dabei konnte nachgewiesen werden, dass die Behandlung mit LCMV eine signifikante Tumorregression bewirkte. Die erhaltenen Ergebnisse sind in Figur 3 grafisch dargestellt.

Die Figur 3 hat die folgende Legende:
Ordinate: Tumor-Durchmesser (cm)
Abszisse: Zeit (Tage)

**2.4** Etwa neun Monate alten LoxP-TAg Mäusen mit spontan entwickelten Leberkarzinomen wurden mit 2 × 10⁶ PFU LCMV intravenös infiziert oder unbehandelt gelassen. Die Tumorknoten (Durchmesser >=3 mm) wurden am Tag 6 (n = 3) und Tag 20 (n = 4-5) makroskopisch quantifiziert.

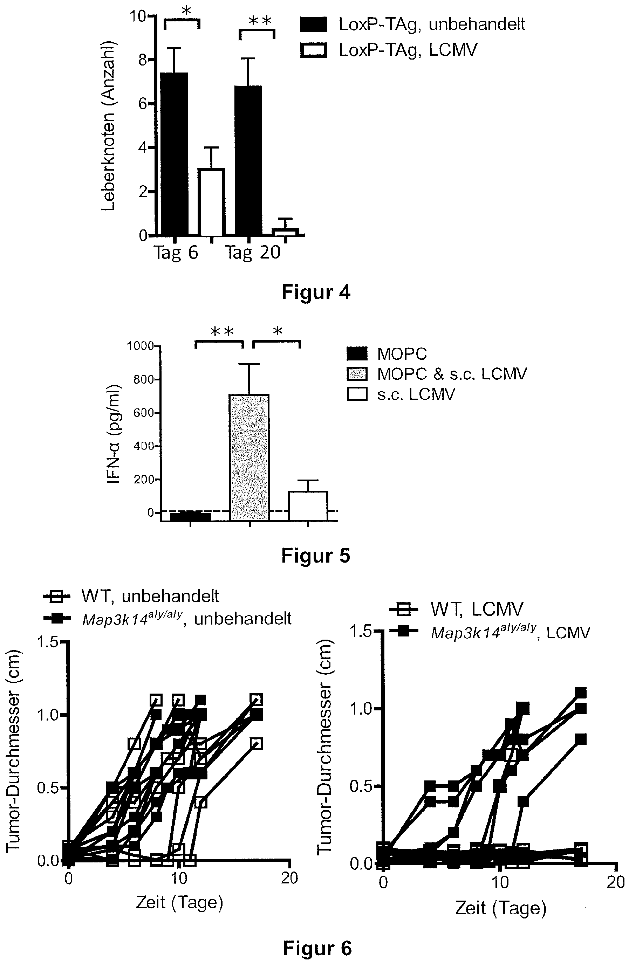

Dabei konnte nachgewiesen werden, dass die Behandlung mit LCMV die Anzahl an karzinomatösen Leberknoten deutlich reduzierte. Die erhaltenen Ergebnisse sind in Figur 4 grafisch dargestellt.

Die Figur 4 hat die folgende Legende:
Ordinate: Leberknoten (Anzahl)
Abszisse: Zeit

**2.5** WT C57BL / 6-Mäuse (n = 4 / Gruppe) wurden subkutan mit 5 × 10⁵ MOPC Zellen (Tag -3) oder LCMV (Stamm WE) 2 × 10⁴ PFU (Tag 0) oder beiden 5 × 10⁵ MOPC Zellen (Tag -3) und 2 × 10⁴ PFU LCMV injiziert (Tag 0). Die Serumproben wurden am Tag 3 gesammelt, und es wurde ein IFN-α-ELISA durchgeführt.

Dabei konnte nachgewiesen werden, dass das LCMV bei Versuchstieren, welchen gleichzeitig Karzinomzellen verabreicht wurden, eine drastisch erhöhte Sezernation von Interferon-y bewirkte. Die erhaltenen Ergebnisse sind in Figur 5 grafisch dargestellt.

Die Figur 5 hat die folgende Legende:
Ordinate: IFN-α (pg/ml)

**2.6** *Map3k14*^{*aly*/*aly*} Mäusen und WT Mäuse wurden mit 5 × 10⁵ MOPC Zellen (Tag 3) behandelt. Eine Gruppe von Mäusen wurde zusätzlich mit 2 × 10⁴ PFU LCMV (Stamm WE) peritumoral behandelt (Tag 0). Das Tumorwachstum wurde beobachtet.

Dabei konnte nachgewiesen werden, dass die Behandlung mit LCMV eine Tumorregression bewirkte. Die erhaltenen Ergebnisse sind in Figur 6 grafisch dargestellt.

Die Figur 6 hat die folgende Legende:
Ordinate: Tumor-Durchmesser (cm)
Abszisse: Zeit (Tage)

**2.7** *Irf3 × Ir7*^{*-*/*-*} Mäusen und WT Mäuse wurden mit 5 × 10⁵ MOPC Zellen (Tag 3) behandelt. Eine Gruppe von Mäusen wurde zusätzlich mit 2 × 10⁴ PFU LCMV (Stamm WE) peritumoral behandelt (Tag 0). Das Tumorwachstum wurde beobachtet.

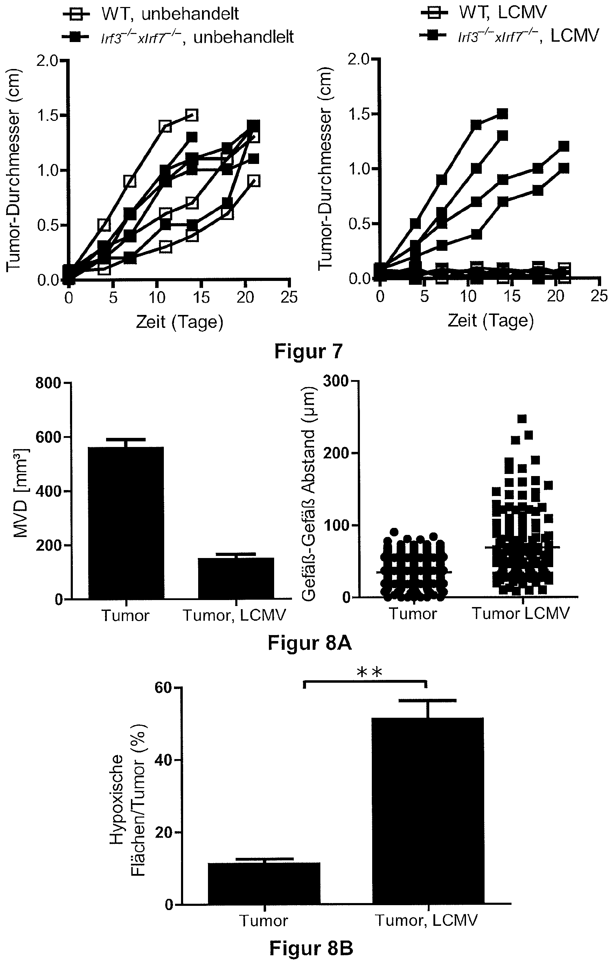

Dabei konnte nachgewiesen werden, dass die Behandlung mit LCMV eine Tumorregression bewirkte. Die erhaltenen Ergebnisse sind in Figur 7 grafisch dargestellt.

Die Figur 7 hat die folgende Legende:
Ordinate: Tumor-Durchmesser (cm)
Abszisse: Zeit (Tage)

**2.8** WT Mäuse wurden mit 5 × 10⁵ MOPC Zellen (Tag 3) behandelt. Eine Gruppe von Mäusen wurde zusätzlich mit 2 × 10⁴ PFU LCMV (Stamm WE) peritumoral behandelt (Tag 0). Am Tag 9 nach Tumorgabe wurden die Tumoren histologisch mit CD31 Färbungen analysiert. Die Gefäßdichte (MVD) und der Gefäß-Gefäß Abstand wurden quantifiziert.

Dabei konnte nachgewiesen werden, dass die Behandlung mit LCMV eine Abnahme der Tumorgefäßdichte bewirkte. Die erhaltenen Ergebnisse sind in Figur 8A grafisch dargestellt.

Die Figur 8A hat die folgenden Legenden:

| Linke Seite: | Rechte Seite: |
|---|---|
| Ordinate: MVD [mm³] | Ordinate: Gefäß-Gefäß Abstand (µm) |
| Abszisse: Tumor/Tumor LCMV | Abszisse: Tumor/Tumor LCMV |

**2.9** WT Mäuse wurden mit 5 × 10⁵ MOPC Zellen (Tag 3) behandelt. Eine Gruppe von Mäusen wurde zusätzlich mit 2 × 10⁴ PFU LCMV (Stamm WE) peritumoral behandelt (Tag 0). Am Tag 9 wurde den Tieren Pimonidazol gespritzt, und anschließend die Tumoren histologisch auf hypoxische Flächen analysiert.

Dabei konnte nachgewiesen werden, dass die Behandlung mit LCMV eine Sauerstoffunterversorgung im Karzinomgewebe bewirkte. Die erhaltenen Ergebnisse sind in Figur 8B grafisch dargestellt.

Die Figur 8B hat die folgende Legende:
Ordinate: Hypoxische Flächen/Tumor (%)
Abszisse: Tumor/Tumor LCMV

**2.10** WT C57BL/6-Mäuse wurden subkutan mit 5 × 10⁵ MOPC Zellen in der rechten Flanke injiziert (Tag 3). Am Tag 0 wurden eine Gruppe von Tieren mit 2 × 10⁴ PFU LCMV (Stamm WE) in die rechte Flanke (ipsilateral), linke Flanke (contralateral) oder intravenös behandelt. Das Tumorwachstum wurde beobachtet.

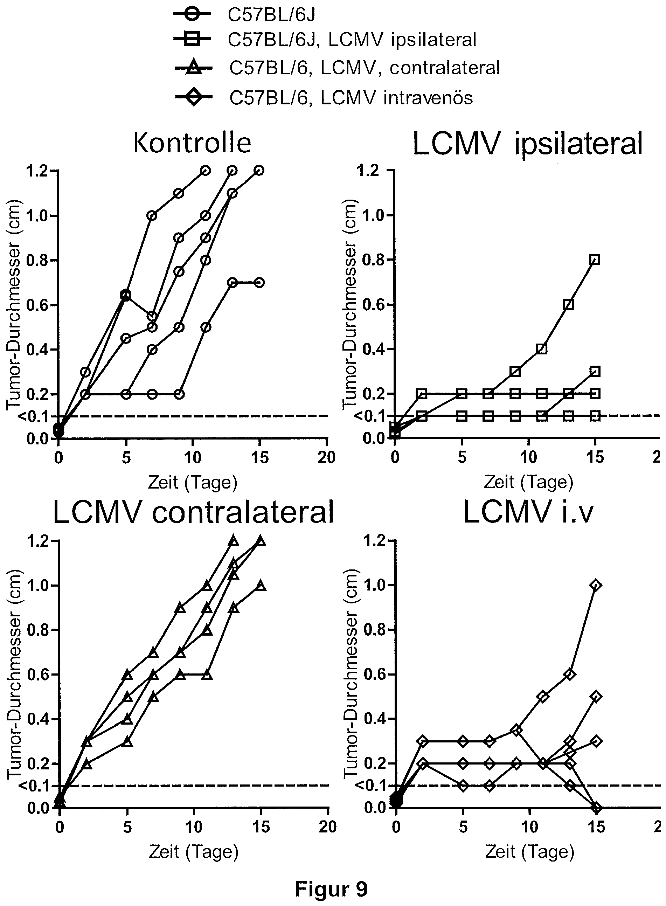

Dabei konnte nachgewiesen werden, dass die Behandlung mit LCMV eine Tumorregression auch bei systemischer Gabe bewirkte. Die erhaltenen Ergebnisse sind in Figur 9 grafisch dargestellt.

Die Figur 9 hat die folgende Legende:
Ordinate: Tumor-Durchmesser (cm)
Abszisse: Zeit (Tage)

**2.11** NOD.SCID-Mäuse wurden mit 5 × 10⁵ A431 Zellen (Tag -3) subkutan injiziert und dann entweder unbehandelt gelassen oder mit 2 × 10⁴ PFU LCMV (Stamm WE) behandelt. Die Tumorgröße (längster Durchmesser) wurde am angegebenen Tage gemessen. Die Mäuse wurden getötet, wenn die Tumorgröße 12 mm erreichte.

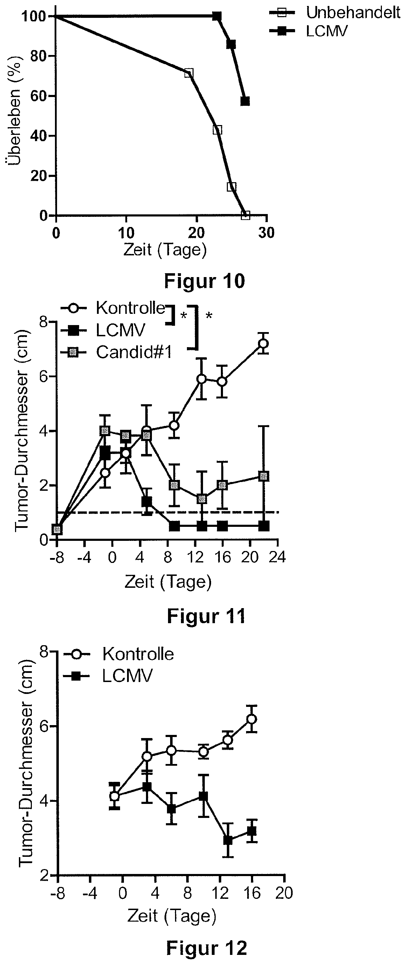

Dabei konnte nachgewiesen werden, dass die Behandlung mit LCMV die Überlebensrate bei den Versuchstieren erhöhte. Die erhaltenen Ergebnisse sind in Figur 10 grafisch dargestellt.

Die Figur 10 hat die folgende Legende:
Ordinate: Überleben (%)
Abszisse: Zeit (Tage)

**2.12** NOD.SCID-Mäuse wurden mit 5 × 10⁵ Sw40 Zellen (Tag 0) behandelt. Eine Gruppe von Mäusen wurde zusätzlich mit 2 × 10⁴ PFU LCMV (Stamm WE) peritumoral oder 2 × 10⁴ PFU Candid#1 peritumoral behandelt (Tag 0). Das Tumorwachstum wurde beobachtet.

Dabei konnte nachgewiesen werden, dass die Behandlung mit LCMV und Candid#1 eine Tumorregression bewirkte. Die erhaltenen Ergebnisse sind in Figur 11 grafisch dargestellt.

Die Figur 11 hat die folgende Legende:
Ordinate: Tumor-Durchmesser (cm)
Abszisse: Zeit (Tage)

**2.13** NOD.SCID-Mäuse wurden mit 5 × 10⁵ Hela Zellen (Tag 0) behandelt. Eine Gruppe von Mäusen wurde zusätzlich mit 2 × 10⁴ PFU LCMV (Stamm WE) peritumoral behandelt (Tag 3). Das Tumorwachstum wurde beobachtet.

Dabei konnte nachgewiesen werden, dass die Behandlung mit LCMV eine Tumorregression bewirkte. Die erhaltenen Ergebnisse sind in Figur 12 grafisch dargestellt.

Die Figur 12 hat die folgende Legende:
Ordinate: Tumor-Durchmesser (cm)
Abszisse: Zeit (Tage)

**2.14** NOD.SCID-Mäuse wurden mit 5 × 10⁵ HepG2 Zellen (Tag -10) behandelt und dann zusätzlich mit oder ohne 2 × 10⁴ PFU Candid #1 peritumoral behandelt (Tag 0). Das Tumorwachstum wurde beobachtet.

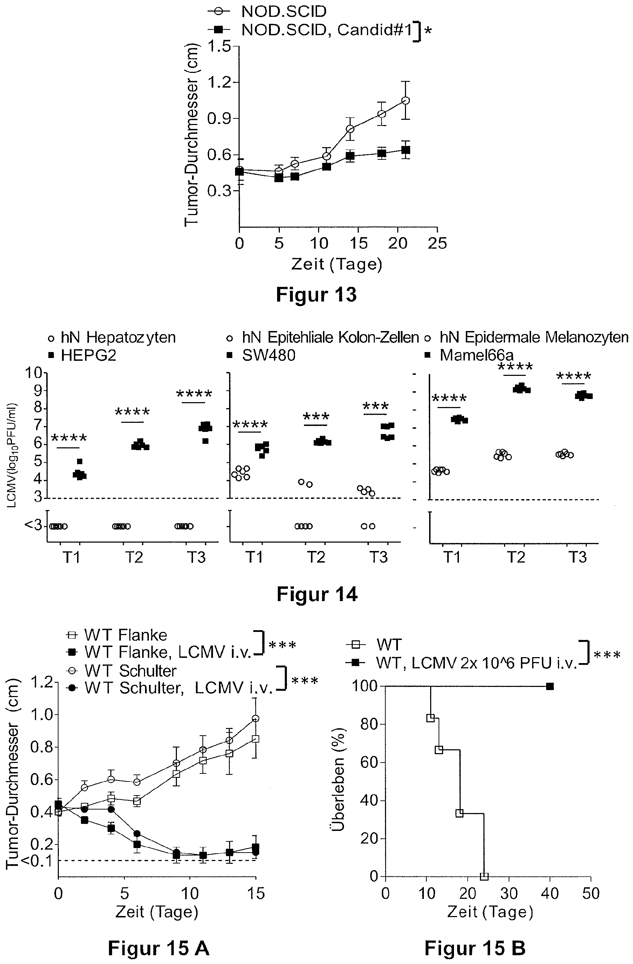

Dabei konnte nachgewiesen werden, dass die Behandlung mit Candid#1 bei dieser Tumorart eine Tumorregression bewirkte. Die erhaltenen Ergebnisse sind in Figur 13 grafisch dargestellt.

Die Figur 13 hat die folgende Legende:
Ordinate: Tumor-Durchmesser (cm)
Abszisse: Zeit (Tage)

**2.15** Primäre humane Zellen (Hepatozyten, Colon Epithelzellen, Melanozyten) und Tumorzellen aus dem selben Ursprungsgewebe wurden mit LCMV infiziert (MOl 1). Virusmenge wurde im Überstand nach 1, 2 und 3 Tagen gemessen.

In diesem Experiment konnte gezeigt werden, das Arenaviren im Vergleich zu gesundem Gewebe, vermehrt in Tumorzellen repliziert.

Die Figur 14 hat die folgende Legende:
Ordinate: Infektiöses Virus im Zellkulturüberstand (Logarithmiert Plaque Forming units)
Abszisse: Zeit (Tage)

**2.16** Tumor-Durchmesser (A) und Überleben (B) von C57BL/6 Mäusen, die eine Metastase in der Schulter und eine Metastase in der Flanke trugen (MOPC Zellen), die unbehandelt gelassen wurden oder mit 2×10⁶ PFU LCMV intravenös behandelt wurden.

In diesem Experiment konnte gezeigt werden, das eine intravenöse Therapie von LCMV sehr effizient auf zwei lokale Metastasen wirkt und somit das Überleben verlängert.

Die Figur 15 A hat die folgende Legende:
Ordinate: Tumor-Durchmesser beider Metastasen (cm)
Abszisse: Zeit (Tage)

Die Figur 15 B hat die folgende Legende:
Ordinate: Überleben in Prozent
Abszisse: Zeit (Tage)

**2.17** Tumor-Durchmesser von C57BL/6 Mäusen, die ein Melanom (B16F10 Zellen) tragen unbehandelt gelassen wurden oder mit 2×10⁴ PFU LCMV intratumoral behandelt wurden.

In diesem Experiment konnte gezeigt werden, das eine lokale Therapie mit LCMV sehr effizient bei Melanom wirkt.

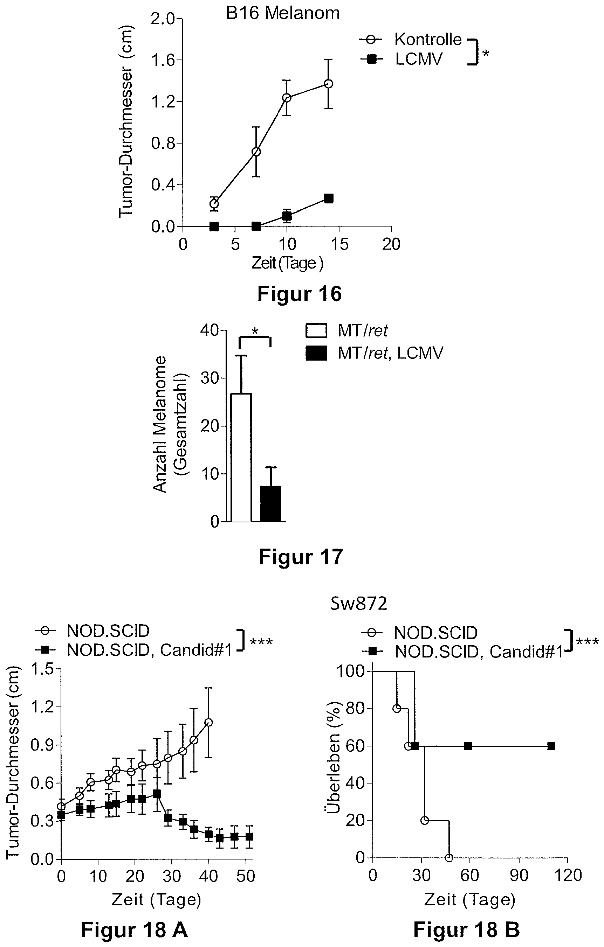

Die Figur 16 hat die folgende Legende:
Ordinate: Tumor-Durchmesser des Melanoms (cm)
Abszisse: Zeit (Tage)

**2.18** Anzahl Melanome von MT/ret Mäusen (entwickeln endogene Melanome), die unbehandelt gelassen wurden oder mit 2×10⁶ PFU LCMV intravenös behandelt wurden.

In diesem Experiment konnte gezeigt werden, das eine systemische Therapie mit LCMV sehr effizient bei Melanom wirkt.

Die Figur 17 hat die folgende Legende:
Ordinate: Anzahl Melanome

**2.19** Tumor-Durchmesser (A) und Überleben (B) von NOD.SCID Mäusen, die eine humanes Fibrosarkom trugen (Sw872 Zellen), die unbehandelt gelassen wurden oder mit 2×10⁶ PFU Candid#1 intratumoral behandelt wurden.

In diesem Experiment konnte gezeigt werden, das Candid#1 auch bei Fibrosarkom sehr effizient wirkt und somit das Überleben verlängert.

Die Figur 18 A hat die folgende Legende:
Ordinate: Tumor-Durchmesser (cm)
Abszisse: Zeit (Tage)

Die Figur 18 B hat die folgende Legende:
Ordinate: Überleben in Prozent
Abszisse: Zeit (Tage)

**2.20** Tumor-Durchmesser (A) und Überleben (B) von NOD.SCID Mäusen, die ein humanes Pharynxkarzinom trugen (FaDu Zellen) und unbehandelt gelassen wurden oder mit 2×10⁶ PFU LCMV intratumoral behandelt wurden.

In diesem Experiment konnte gezeigt werden, das LCMV auch bei humanem Pharynxkarzinom sehr effizient wirkt und somit das Überleben verlängert.

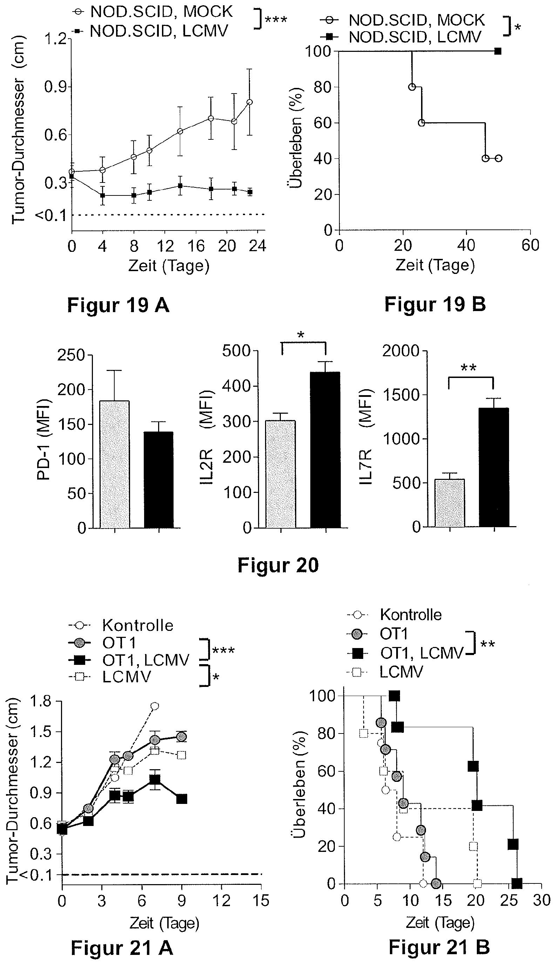

Die Figur 19 A hat die folgende Legende:
Ordinate: Tumor-Durchmesser (cm)
Abszisse: Zeit (Tage)

Die Figur 19 B hat die folgende Legende:
Ordinate: Überleben in Prozent
Abszisse: Zeit (Tage)

**2.21** Expression von Rezeptoren auf tumorspezifischen T Zellen (PD-1, IL2 Rezeptor, IL7 Rezeptor), die die Funktion von T Zellen beeinflussen. Tumor-spezifische T Zellen stammen aus dem Blut von Mäusen mit B16F10 Tumoren, die zusätzlich mit oder ohne LCMV intratumoral behandelt wurden.

In diesem Experiment konnte gezeigt werden, das LCMV die tumor-spezifischen T Zellen positiv beeinflußt.

Die Figur 20 hat die folgende Legende:
Ordinate: Stärke der Expression der unterschiedlichen Rezeptoren (Mittlere Fluoreszenz Intensität)

**2.22** Tumor-Durchmesser (A) und Überleben (B) von C57BL/6 Mäusen, die ein murines subkutanes Lymphom trugen (EL4 Zellen) mit oder ohne tumor-spezifischen T Zellen behandelt wurden (OT1-Zellen) und zusätzlich mit oder ohne LCMV intratumoral behandelt wurden (2×10⁶ PFU).

In diesem Experiment konnte gezeigt werden, das LCMV synergistisch mit einer T Zelltherapie wirkt.

Die Figur 21 A hat die folgende Legende:
Ordinate: Tumor-Durchmesser (cm)
Abszisse: Zeit (Tage)

Die Figur 21 B hat die folgende Legende:
Ordinate: Überleben in Prozent
Abszisse: Zeit (Tage)

**2.23** Überleben von C57BU6 Mäusen und PD-1 defizienten Mäusen (*Pdcd1*^{-/-}Mäuse), die ein murines Pharynxkarzinom trugen (MOPC Zellen) und mit LCMV intratumoral behandelt wurden (2×10⁴ PFU).

In diesem Experiment konnte gezeigt werden, das LCMV synergistisch mit einer PD-1 Blockade wirkt.

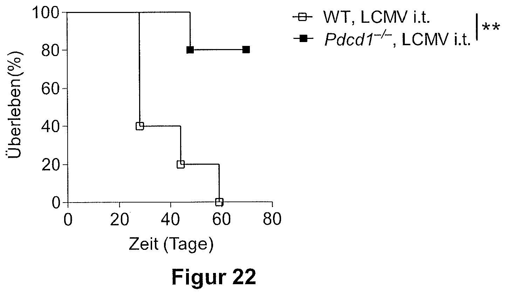

Die Figur 22 hat die folgende Legende:
Ordinate: Überleben in Prozent
Abszisse: Zeit (Tage)

Die in der allgemeinen Beschreibung erwähnten Nukleinsäuresequenzen SEQ ID No. 1 bis SEQ ID No. 6 entsprechen den im nachfolgenden Sequenzprotokoll offenbarten Nukleinsäuresequenzen.

## Patentansprüche

1. Arenavirus zur Anwendung bei der Behandlung eines Tumors beim Menschen, wobei eine Tumorregression bewirkt wird, wobei das Arenavirus frei von genomischer Fremd-RNA ist, wobei das Arenavirus Lymphozytäre-Choriomeningitis-Virus, WE-Stamm, ist.

2. Arenavirus zur Anwendung nach Anspruch 1, wobei die Behandlung die Aktivierung von angeborenen und adaptiven Immunzellen umfasst, wodurch eine Tumorregression bewirkt wird.

3. Arenavirus zur Anwendung nach Anspruch 1 oder 2, wobei der Tumor ein Karzinom, Melanom, Blastom, Lymphom oder Sarkom ist.

4. Medikament zur Anwendung bei der Behandlung eines Tumors, beim Menschen wobei das Medikament ein Arenavirus nach einem der vorhergehenden Ansprüche sowie vorzugsweise einen pharmazeutisch verträglichen Träger aufweist.

## Claims

1. An arenavirus for use in the treatment of a tumor in a human, wherein tumor regression is effected, wherein the arenavirus is free of foreign genomic RNA, wherein the arenavirus is lymphocytic choriomeningitis virus, WE strain.

2. The arenavirus for the use of claim 1, wherein the treatment comprises activating innate and adaptive immune cells, thereby causing tumor regression.

3. The arenavirus for the use of claim 1 or 2, wherein the tumor is a carcinoma, melanoma, blastoma, lymphoma or sarcoma.

4. A medicament for use in the treatment of a tumor, in a human, wherein the medicament comprises an arenavirus of any one of the preceding claims and preferably a pharmaceutically acceptable carrier.

## Revendications

1. Arenavirus destiné à être utilisé dans le traitement d'une tumeur chez l'homme, provoquant ainsi une régression de la tumeur, ledit arenavirus étant exempt d'ARN génomique étranger, ledit arenavirus étant le virus de la chorioméningite lymphocytaire, souche WE.

2. Arenavirus pour utilisation selon la revendication 1, dans lequel le traitement comprend l'activation de cellules immunitaires innées et adaptatives, provoquant ainsi une régression tumorale.

3. Arenavirus pour utilisation selon la revendication 1 ou 2, dans lequel la tumeur est un carcinome, un mélanome, un blastome, un lymphome ou un sarcome.

4. Médicament destiné à être utilisé dans le traitement d'une tumeur chez l'homme, ledit médicament comprenant un arénavirus selon l'une quelconque des revendications précédentes et, de préférence, un support pharmaceutiquement acceptable.
